(19) Europäisches Patentamt
European Patent Office
Office européen des brevets

(11) EP 2 149 612 A1

(12) EUROPEAN PATENT APPLICATION

(43) Date of publication:
03.02.2010 Bulletin 2010/05

(51) Int Cl.:
*C12Q 1/68* *(2006.01)*

(21) Application number: 08161361.4

(22) Date of filing: 29.07.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicant: **Merck Serono SA**
**1267 Coinsins, Vaud (CH)**

(72) Inventors:
- **Monnet, Emmanuel**
  **74160 Saint Julien en Genevois (FR)**
- **Bouzekri, Nourdine**
  **74100 Annemasse (FR)**

(74) Representative: **Merck Serono SA - Geneva Intellectual Property**
**9, chemin des Mines**
**1202 Geneva (CH)**

Remarks:
A request for correction of part of the description (removal of blank page numbered 38) has been filed pursuant to Rule 139 EPC.

# (54) Genetic markers of response to efalizumab

(57) The invention relates to genetic markers that are associated with a particular clinical response to treatment with an inhibitor of LFA-1 in individuals afflicted with psoriasis. The genetic markers can be used to identify and/or select individuals afflicted with psoriasis that are more likely to exhibit a particular clinical response to treatment with an inhibitor of LFA-1.

EP 2 149 612 A1

**Description**

FIELD OF THE INVENTION

The present invention relates to pharmacogenetics. More specifically, the invention relates to genetic markers that are associated with clinical response to efalizumab when used for the treatment of psoriasis.

BACKGROUND OF THE INVENTION

Psoriasis is a chronic skin disorder with a worldwide prevalence of approximately 2%. Plaque psoriasis is the most common form of psoriasis, affecting about 80% of cases, and is characterized by demarcated, erythematous, scaling plaques typically affecting the elbows, knees, scalp, sacrum, intergluteal cleft, and genitalia. Patients also often experience pain, itching, burning, and bleeding lesions. Furthermore, between 5 and 42% of patients with psoriasis will have psoriatic arthritis, a potentially disabling joint disease. Although mortality does not appear to be increased in patients with psoriasis, the negative impact on quality of life in affected individuals is similar to that in patients with coronary heart disease or chronic obstructive pulmonary disease.

Although the disease occurs in all age groups, it primarily affects adults. It appears about equally in males and females. Psoriasis occurs when skin cells quickly rise from their origin below the surface of the skin and pile up on the surface before they have a chance to mature. Psoriasis is considered mild if it affects less than 5% of the surface of the body, moderate if 5-30% of the skin is involved, and severe if the disease affects more than 30% of the body surface.

There are a number of psoriatic subtypes which describe the area of involvement, for example flexural psoriasis (where the skin lesions occur on flexor surfaces such as the groin), or the pattern of cutaneous change, for example psoriasis annularis (psoriasis with lesions occurring in ring shaped patches), or the type of cutaneous lesion, for example pustular psoriasis (where pustules predominate rather than papules, plaques or macules).

Psoriatic arthritis, an erosive and usually asymmetrical oligoarthritis, may occur with this chronic recurrent papulo-squamous skin disorder.

Because psoriasis is currently incurable, the goal of treatment is to provide symptomatic relief and improved quality of life. Common treatment modalities for plaque psoriasis include topical treatments (e.g. emollients, keratolytic agents, and/or corticosteroids) or systemic therapy with ciclosporin, methotrexate, acitretin, or psoralens plus ultraviolet A therapy. However, although beneficial for some patients with plaque psoriasis, a number of these treatments are not always well tolerated, particularly when used long term.

A greater understanding of the pathogenesis of psoriasis has led to the development of more specific treatment modalities that have good efficacy without the adverse effects of the more commonly used therapies. One such development is efalizumab, a humanized form of a murine antibody against CD11a ($\alpha_L$), the $\alpha$-subunit of lymphocyte function-associated antigen-1 (LFA-1). LFA-1 is a T-cell surface molecule in T-cell activation, cutaneous T-cell trafficking, and T-cell adhesion that consists of an $\alpha$-subunit CD11a and a $\beta$-subunit CD18 ($\beta_2$). A synonym for LFA-1 is also integrin $\alpha_L/\beta_2$

Psoriasis treatment with efalizumab is provided to patients without predictive information on the response; i.e. knowledge on whether the treatment will be highly effective, moderately effective or show only little effect. Currently only a portion of the treated patient population will exhibit a very good response to treatment according to the Physician Global Assessment (PGA) and Psoriasis Area and Severity Index (PASI) scores with efalizumab after 2 to 3 years of treatment.

Here we describe for the first time genetic markers that are associated with the quality of the clinical response to treatment of psoriasis with efalizumab. Such markers are useful for identifying through genetic screening prior to the treatment subgroups of patients that are more likely to exhibit a particular response to treatment with efalizumab, such as a very good clinical response to treatment with efalizumab. Knowledge on the type of clinical response of a patient to treatment can be used to optimize therapy or select therapy, such selecting treatment with efalizumab as a first line therapy.

SUMMARY OF THE INVENTION

A first aspect of the invention is a method of genotyping an individual afflicted with psoriasis comprising detecting the presence or absence of a particular allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual.

A second aspect of the invention is a kit comprising means for detecting the presence or absence of a particular allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual and instructions for use.

A third aspect of the invention is a method of diagnosis to identify an individual afflicted with psoriasis that is more likely to exhibit a particular response to treatment with an inhibitor of LFA-1 comprising detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual.

A fourth aspect of the invention is a method of treating psoriasis comprising detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of an individual afflicted with psoriasis, selecting an individual afflicted with psoriasis based on the allele of said marker that that is detected in the individual, and administering to said selected individual a therapeutically effective amount of an inhibitor of LFA-1.

A fifth aspect of the invention is an inhibitor of LFA-1 for use in the treatment of psoriasis **characterized in that** the treatment comprises detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of an individual afflicted with psoriasis, selecting an individual afflicted with psoriasis based on the allele of said marker that is detected in the individual, and administering to said selected individual a therapeutically effective amount of an inhibitor of LFA-1.

A sixth aspect of the invention is an inhibitor of LFA-1 for use in the treatment of psoriasis in individuals afflicted with psoriasis **characterized in that** the individual has one allele or any combination of the allele(s) or genotypes selected from the group consisting of thymine ("T") for the marker SNP_A-2040233 (rs4043263), adenine ("A") for the marker SNP_A-1978609 (rs17501861), cytosine ("C") for the marker SNP_A-1986846 (rs9294506), adenine ("A") for the marker SNP_A-2223123 (rs6485472), thymine ("T") for the marker SNP_A-2085239 (rs11251116), guanine ("G") for the marker SNP_A-4269573 (rs11037653), guanine ("G") for the marker SNP_A-2010642 (rs4620711), guanine ("G") for the marker SNP_A-4260850 (rs10838166), cytosine ("C") for the marker SNP_A-4239787 (rs4573056), adenine ("A") for the marker SNP_A-2213961 (rs10755496) or cytosine ("C") for the marker SNP_A-2299781 (rs7742750).

A preferred inhibitor of LFA-1 is an inhibitor of CD11a. Preferably the inhibitor of LFA-1 or CD11a is a peptide or protein, more preferably an antibody or antigen binding fragment thereof, even more preferred an antibody or antigen binding fragment thereof that binds specifically to CD11a, and most preferred the inhibitor of LFA-1 is efalizumab, an antigen binding fragment thereof or a derivative of efalizumab comprising all three heavy chain CDRs and/or all three light chain CDRs or at least one CDR of efalizumab.

## BRIEF DESCRIPTION OF THE FIGURES

Figure 1: The PASI score of an individual afflicted with psoriasis can be assessed by the physician using a questionnaire as shown in Fig. 1.

Figure 2: The distribution of allelic (nucleotide T or C) and genotypic (nucleotides TT, CT or CC) forms of marker SNP_A-2040233 in psoriasis patients grouped according to the PGA response definition is shown in Fig. 2(a). The number of patients with a particular allele or genotype and achieving a particular clinical response to treatment with efalizumab is provided as well as the number of all patients irrespective of the allele or genotype ("total"). The statistical characteristics from the whole genome scan for marker SNP_A-2040233 is shown in Fig. 2(b).

Figure 3: The distribution of allelic (nucleotide T or A) and genotypic (nucleotides TT, AT or AA) forms of marker SNP_A-1978609 in psoriasis patients grouped according to the PGA response definition is shown in Fig. 3(a). The number of patients with a particular allele or genotype and achieving a particular clinical response to treatment with efalizumab is provided as well as the number of all patients irrespective of the allele or genotype ("total"). The statistical characteristics from the whole genome scan for marker SNP_A-1978609 is shown in Fig. 3(b).

Figure 4: The distribution of allelic (nucleotide G or C) and genotypic (nucleotides GG, GC or CC) forms of marker SNP_A-1986846 in psoriasis patients grouped according to the PASI response definition is shown in Fig. 4(a). The number of patients with a particular allele or genotype and achieving a particular clinical response to treatment with efalizumab is provided as well as the number of all patients irrespective of the allele or genotype ("total"). The statistical characteristics from the whole genome scan for marker SNP_A-1986846 is shown in Fig. 4(b).

Figure 5: The distribution of allelic (nucleotide C or A) and genotypic (nucleotides CC, AC or AA) forms of marker SNP_A-2223123 in psoriasis patients grouped according to the PASI response definition is shown in Fig. 5(a). The number of patients with a particular allele or genotype and achieving a particular clinical response to treatment with efalizumab is provided as well as the number of all patients irrespective of the allele or genotype ("total"). The statistical characteristics from the whole genome scan for marker SNP_A-2223123 is shown in Fig. 5(b).

Figure 6: The distribution of allelic (nucleotide T or G) and genotypic (nucleotides TT, GT or GG) forms of marker SNP_A-2085239 in psoriasis patients grouped according to the PASI response definition is shown in Fig. 6(a). The number of patients with a particular allele or genotype and achieving a particular clinical response to treatment with efalizumab is provided as well as the number of all patients irrespective of the allele or genotype ("total"). The statistical characteristics from the whole genome scan for marker SNP_A-2085239 is shown in Fig. 6(b).

## DETAILED DESCRIPTION OF THE INVENTION

The invention is based on genetic markers in individuals afflicted with psoriasis. It has now been identified by the present inventors that surprisingly a particular allele or genotype of certain genetic markers as described herein (see Table 2) is associated with a particular clinical response to treatment of psoriasis with an inhibitor of LFA-1. It has further been found by the present inventors that individuals afflicted with psoriasis that have said allele or genotype of said genetic markers are more likely to exhibit a particular clinical response to the treatment of psoriasis with efalizumab, such as a very good response, a response or a partial response according to the Physician Global Assessment (PGA) or the Psoriasis Area and Severity Index (PASI) score. Consequently, the alleles or genotypes of the genetic markers found to be associated with the clinical response to treatment of psoriasis with efalizumab can be detected in individuals afflicted with psoriasis, and individuals can be selected for treatment with efalizumab based on the allele or genotype of the genetic marker that they have.

Five genetic markers were identified that are particularly useful for selecting individuals afflicted by psoriasis that are likely to exhibit a particular clinical response to treatment with efalizumab: SNP_A-2040233 (rs4043263), SNP_A-1978609 (rs17501861), SNP_A-1986846 (rs9294506), SNP_A-2223123 (rs6485472) and SNP_A-2085239 (rs11251116). The above genetic markers of the invention are single nucleotide polymorphisms (SNPs) and further described in Tables 1 and 2. These genetic markers are present in approximately 5 to 20% of the psoriasis population.

The three genetic markers SNP_A-4269573 (rs11037653), SNP_A-2010642 (rs4620711) and SNP_A-4260850 (rs10838166) were found to be in linkage disequilibrium with marker SNP_A-2223123 (rs6485472). The three genetic markers SNP_A-4239787 (rs4573056), SNP_A-2213961 (rs10755496) and SNP_A-2299781 (rs7742750) were found to be in linkage disequilibrium with marker SNP_A-1986846 (rs9294506). Consequently, the genetic markers SNP_A-4269573 (rs11037653), SNP_A-2010642 (rs4620711), SNP_A-4260850 (rs10838166), SNP_A-4239787 (rs4573056), SNP_A-2213961 (rs10755496) and SNP_A-2299781 (rs7742750) can also be used for selecting individuals afflicted by psoriasis that are likely to exhibit a particular clinical response to treatment with efalizumab.

All genetic markers of the invention are markers of likelihood (i.e. no marker was 100% associated with a particular clinical response), with a relative likelihood of 1.4-2.0 and fairly narrow 95% confidence intervals. The best response rate to treatment with efalizumab in an individual afflicted with psoriasis observed was approximately 95% of responders (based on PGA response) that represent 13% of the population (marker SNP_A-1978609), and approximately 80% of responders (based on PASI75) that represent 4% of the population (marker SNP_A-2085239). The worst response rates observed were approximately 65% of responders (based on PGA response) that represent 15% of the population (marker SNP_A-2040233) and approximately 25% of responders (based on PASI 75) that represent 13% of the population (marker SNP_A-2223123).

In addition, it was noted that two genetic markers of the invention are located in or close to the functional structure of two genes. Marker SNP_A-2040233 is close to the gene ADAM11 also called metallo-peptidase domain 11 that is involved in cell adhesion and trafficking. Marker SNP_A-2223123 is in the gene HSD17B12 also called HydroxySteroid (17-beta) Dehydrogenase that is involved in steroid metabolism. The other three genetic markers of the invention are not part of or close to any coding or non-coding parts of known genes.

Surprisingly, it has been found by the present inventors that the allele thymine ("T") of the marker SNP_A-2040233 (rs4043263) is associated with a good or better response to treatment with efalizumab in individuals afflicted with psoriasis, wherein the likelihood of exhibiting a good or better response to treatment with efalizumab is higher in individuals afflicted with psoriasis that are homozygous for the allele thymine ("TT") as compared to individuals afflicted with psoriasis that are heterozygous for the allele thymine ("CT").

Surprisingly, it has been found by the present inventors that the allele adenine ("A") of the marker SNP_A-1978609 (rs17501861) is associated with a good or better response to treatment with efalizumab in individuals afflicted with psoriasis, wherein the likelihood of exhibiting a good or better response to treatment with efalizumab is higher in individuals afflicted with psoriasis that are homozygous for the allele adenine ("AA") as compared to individuals afflicted with psoriasis that are heterozygous for the allele adenine ("AT").

Surprisingly, it has been found by the present inventors that the allele cytosine ("C") of the marker SNP_A-1986846 (rs9294506) is associated with a response or partial response to treatment with efalizumab in individuals afflicted with psoriasis, wherein the likelihood of exhibiting a response or partial response to treatment with efalizumab is higher in individuals afflicted with psoriasis that are homozygous for the allele cytosine ("CC") as compared to individuals afflicted with psoriasis that are heterozygous for the allele cytosine ("CG").

Surprisingly, it has been found by the present inventors that the allele adenine ("A") of the marker SNP_A-2223123 (rs6485472) is associated with a response to treatment with efalizumab in individuals afflicted with psoriasis, wherein the likelihood of exhibiting a response to treatment with efalizumab is higher in individuals afflicted with psoriasis that are homozygous for the allele adenine ("AA") as compared to individuals afflicted with psoriasis that are heterozygous for the allele adenine ("AC").

Surprisingly, it has been found by the present inventors that the allele thymine ("T") of the marker SNP_A-2085239 (rs11251116) is associated with a response to treatment with efalizumab in individuals afflicted with psoriasis, wherein the likelihood of exhibiting a response to treatment with efalizumab is higher in individuals afflicted with psoriasis that are homozygous for the allele thymine ("TT") as compared to individuals afflicted with psoriasis that are heterozygous for the allele thymine ("GT").

The three genetic markers SNP_A-4269573 (rs11037653), SNP_A-2010642 (rs4620711) and SNP_A-4260850 (rs10838166) were found to be in linkage disequilibrium with marker SNP_A-2223123 (rs6485472). The three genetic markers SNP_A-4239787 (rs4573056), SNP_A-2213961 (rs10755496) and SNP_A-2299781 (rs7742750) were found to be in linkage disequilibrium with marker SNP_A-1986846 (rs9294506).

Accordingly, a first aspect of the invention is a method of genotyping an individual afflicted with psoriasis comprising detecting the presence or absence of a particular allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual.

A first embodiment of the first aspect of the invention is a method of genotyping an individual afflicted with psoriasis comprising detecting the presence or absence of the allele thymine ("T") of the marker SNP_A-2040233 (rs4043263) on one chromosome or both of paired chromosomes in a biological sample of the individual.

A second embodiment of the first aspect of the invention is a method of genotyping an individual afflicted with psoriasis comprising detecting the presence or absence of the allele adenine ("A") of the marker SNP_A-1978609 (rs17501861) on one chromosome or both of paired chromosomes in a biological sample of the individual.

A third embodiment of the first aspect of the invention is a method of genotyping an individual afflicted with psoriasis comprising detecting the presence or absence of the allele cytosine ("C") of the marker SNP_A-1986846 (rs9294506) on one chromosome or both of paired chromosomes in a biological sample of the individual.

A fourth embodiment of the first aspect of the invention is a method of genotyping an individual afflicted with psoriasis comprising detecting the presence or absence of the allele adenine ("A") of the marker SNP_A-2223123 (rs6485472) on one chromosome or both of paired chromosomes in a biological sample of the individual.

A fifth embodiment of the first aspect of the invention is a method of genotyping an individual afflicted with psoriasis comprising detecting the presence or absence of the allele thymine ("T") of the marker SNP_A-2085239 (rs11251116) on one chromosome or both of paired chromosomes in a biological sample of the individual.

A sixth embodiment of the first aspect of the invention is a method of genotyping an individual afflicted with psoriasis comprising detecting the presence or absence of the allele guanine ("G") of the marker SNP_A-4269573 (rs11037653) on one chromosome or both of paired chromosomes in a biological sample of the individual.

A seventh embodiment of the first aspect of the invention is a method of genotyping an individual afflicted with psoriasis comprising detecting the presence or absence of the allele guanine ("G") of the marker SNP_A-2010642 (rs4620711) on one chromosome or both of paired chromosomes in a biological sample of the individual.

A eighth embodiment of the first aspect of the invention is a method of genotyping an individual afflicted with psoriasis comprising detecting the presence or absence of the allele guanine ("G") of the marker SNP_A-4260850 (rs10838166) on one chromosome or both of paired chromosomes in a biological sample of the individual.

A ninth embodiment of the first aspect of the invention is a method of genotyping an individual afflicted with psoriasis comprising detecting the presence or absence of the allele cytosine ("C") of the marker SNP_A-4239787 (rs4573056) on one chromosome or both of paired chromosomes in a biological sample of the individual.

A tenth embodiment of the first aspect of the invention is a method of genotyping an individual afflicted with psoriasis comprising detecting the presence or absence of the allele adenine ("A") of the marker SNP_A-2213961 (rs10755496) on one chromosome or both of paired chromosomes in a biological sample of the individual.

A eleventh embodiment of the first aspect of the invention is a method of genotyping an individual afflicted with psoriasis comprising detecting the presence or absence of the allele cytosine ("C") of the marker SNP_A-2299781 (rs7742750) on one chromosome or both of paired chromosomes in a biological sample of the individual.

A twelfth embodiment of the first aspect of the invention is a method according the first, second, third, fourth, fifth, sixth, seventh, eighth, ninth, tenth, eleventh embodiment of the first aspect of the invention, wherein the presence of absence of the alleles of at least two of the genetic markers are detected.

A second aspect of the invention is a kit comprising means for detecting the presence or absence of a particular allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual, and instructions for use.

A first embodiment of the second aspect of the invention is a kit comprising means for genotyping an individual afflicted with psoriasis according to any of the first, second, third, fourth, fifth, sixth, seventh, eighth, ninth, tenth, eleventh embodiment of the first aspect of the invention.

A second embodiment of the second aspect of the invention is a kit comprising a specific primer or probe, such as without limitation an oligonucleotide, for detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual, and instructions for use. A particularly preferred embodiment of the invention is a kit according to the second embodiment of the second aspect of the invention wherein the specific primer or probe is for detecting the presence or absence of the allele thymine ("T") of the marker SNP_A-2040233 (rs4043263), the allele adenine ("A") of the marker SNP_A-1978609 (rs17501861), the allele cytosine ("C") of the marker SNP_A-1986846 (rs9294506), the allele adenine ("A") of the marker SNP_A-2223123 (rs6485472), the allele thymine ("T") of the marker SNP_A-2085239 (rs11251116), the allele guanine ("G") for the marker SNP_A-4269573 (rs11037653), the allele guanine ("G") for the marker SNP_A-2010642 (rs4620711), the allele guanine ("G") for the marker SNP_A-4260850 (rs10838166), the allele cytosine ("C") for the marker SNP_A-4239787 (rs4573056), the allele adenine ("A") for the marker SNP_A-2213961 (rs10755496) or the allele cytosine ("C") for the marker SNP_A-2299781 (rs7742750).

A third aspect of the invention is a method of diagnosis to identify an individual afflicted with psoriasis that is more likely to exhibit a particular response to treatment with an inhibitor of LFA-1 comprising detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of the individual. A particularly preferred embodiment of the invention is the method of diagnosis according the third aspect of the invention wherein the allele the presence or absence of which is detected is thymine ("T") for the marker SNP_A-2040233 (rs4043263), adenine ("A") for the marker SNP_A-1978609 (rs17501861), cytosine ("C") for the marker SNP_A-1986846 (rs9294506), adenine ("A") for the marker SNP_A-2223123 (rs6485472), thymine ("T") for the marker SNP_A-2085239 (rs11251116),, guanine ("G") for the marker SNP_A-4269573 (rs11037653), guanine ("G") for the marker SNP_A-2010642 (rs4620711), guanine ("G") for the marker SNP_A-4260850 (rs10838166), cytosine ("C") for the marker SNP_A-4239787 (rs4573056), adenine ("A") for the marker SNP_A-2213961 (rs10755496) or cytosine ("C") for the marker SNP_A-2299781 (rs7742750) wherein the presence of the respective alleles above indicates that the individual is more likely to exhibit a particular response to treatment with an inhibitor of LFA-1, such as a very good response, a response or a partial response preferably assessed according to the Physician Global Assessment (PGA) score.

A first embodiment of the fourth aspect of the invention is a method of diagnosis to identify an individual afflicted with psoriasis that is more likely to exhibit a particular response to treatment with an inhibitor of LFA-1 comprising genotyping an individual afflicted with psoriasis according the any of the first, second, third, fourth, fifth, sixth, seventh, eighth, ninth, tenth, eleventh embodiment of the first aspect of the invention.

A second embodiment of the third aspect of the invention is the method of diagnosis according to the third aspect of the invention wherein the individual identified is more likely to exhibit a very good response, a response or a partial response to the treatment of psoriasis with an inhibitor of LFA-1 as assessed by the PGA score.

A fourth aspect of the invention is a method of treating psoriasis comprising detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of an individual afflicted with psoriasis, selecting an individual afflicted with psoriasis based on the allele of said marker that is detected in the individual, and administering to said selected individual a therapeutically effective amount of an inhibitor of LFA-1.

A first embodiment of the fourth aspect of the invention is a method of treating psoriasis comprising genotyping an individual afflicted with psoriasis according the any of the first, second, third, fourth, fifth, sixth, seventh, eighth, ninth, tenth, eleventh embodiment of the first aspect of the invention, selecting an individual afflicted with psoriasis based on the allele of said marker that is detected in the individual, and administering to said selected individual a therapeutically effective amount of an inhibitor of LFA-1.

A second embodiment of the fourth aspect of the invention is a method of treating psoriasis comprising detecting the presence or absence of the allele thymine ("T") of the marker SNP_A-2040233 (rs4043263) on one chromosome or both of paired chromosomes in a biological sample of an individual afflicted with psoriasis, selecting an individual afflicted with psoriasis detected with the allele thymine ("T") of said marker, and administering to said selected individual a therapeutically effective amount of an inhibitor of LFA-1, wherein the individual selected is either heterozygous or homozygous for said allele but preferentially homozygous.

A third embodiment of the fourth aspect of the invention is a method of treating psoriasis comprising detecting the presence or absence of the allele adenine ("A") of the genetic marker SNP_A-1978609 (rs17501861) on one chromosome or both of paired chromosomes in a biological sample of an individual afflicted with psoriasis, selecting an individual afflicted with psoriasis detected with the allele adenine ("A") of said marker, and administering to said selected individual a therapeutically effective amount of an inhibitor of LFA-1, wherein the individual selected is either heterozygous or homozygous for said allele but preferentially homozygous.

A fourth embodiment of the fourth aspect of the invention is a method of treating psoriasis comprising detecting the presence or absence of the allele cytosine ("C") of the genetic marker SNP_A-1986846 (rs9294506) on one chromosome or both of paired chromosomes in a biological sample of an individual afflicted with psoriasis, selecting an individual afflicted with psoriasis detected with the allele cytosine ("C") of said marker, and administering to said selected individual a therapeutically effective amount of an inhibitor of LFA-1, wherein the individual selected is either heterozygous or homozygous for said allele but preferentially homozygous.

A fifth embodiment of the fourth aspect of the invention is a method of treating psoriasis comprising detecting the presence or absence of the allele adenine ("A") of the genetic marker SNP_A-2223123 (rs6485472) on one chromosome or both of paired chromosomes in a biological sample of an individual afflicted with psoriasis, selecting an individual afflicted with psoriasis detected with the allele adenine ("A") of said marker, and administering to said selected individual a therapeutically effective amount of an inhibitor of LFA-1, wherein the individual selected is either heterozygous or homozygous for said allele but preferentially homozygous.

A sixth embodiment of the fourth aspect of the invention is a method of treating psoriasis comprising detecting the presence or absence of the allele thymine ("T") of the genetic marker SNP_A-2085239 (rs11251116) on one chromosome or both of paired chromosomes in a biological sample of an individual afflicted with psoriasis, selecting an individual afflicted with psoriasis detected with the allele thymine ("T") of said marker, and administering to said selected individual a therapeutically effective amount of an inhibitor of LFA-1, wherein the individual selected is either heterozygous or homozygous for said allele but preferentially homozygous.

A seventh embodiment of the fourth aspect of the invention is a method of treating psoriasis comprising detecting the presence or absence of the allele guanine ("G") of the genetic marker SNP_A-4269573 (rs11037653) on one chromosome or both of paired chromosomes in a biological sample of an individual afflicted with psoriasis, selecting an individual afflicted with psoriasis detected with the allele guanine ("G") of said marker, and administering to said selected individual a therapeutically effective amount of an inhibitor of LFA-1, wherein the individual selected is either heterozygous or homozygous for said allele but preferentially homozygous.

A eighth embodiment of the fourth aspect of the invention is a method of treating psoriasis comprising detecting the presence or absence of the allele guanine ("G") of the genetic marker SNP_A-2010642 (rs4620711) on one chromosome or both of paired chromosomes in a biological sample of an individual afflicted with psoriasis, selecting an individual afflicted with psoriasis detected with the allele guanine ("G") of said marker, and administering to said selected individual a therapeutically effective amount of an inhibitor of LFA-1, wherein the individual selected is either heterozygous or homozygous for said allele but preferentially homozygous.

A ninth embodiment of the fourth aspect of the invention is a method of treating psoriasis comprising detecting the presence or absence of the allele guanine ("G") of the genetic marker SNP_A-4260850 (rs10838166) on one chromosome or both of paired chromosomes in a biological sample of an individual afflicted with psoriasis, selecting an individual afflicted with psoriasis detected with the allele guanine ("G") of said marker, and administering to said selected individual a therapeutically effective amount of an inhibitor of LFA-1, wherein the individual selected is either heterozygous or homozygous for said allele but preferentially homozygous.

A tenth embodiment of the fourth aspect of the invention is a method of treating psoriasis comprising detecting the presence or absence of the allele cytosine ("C") of the genetic marker SNP_A-4239787 (rs4573056) on one chromosome or both of paired chromosomes in a biological sample of an individual afflicted with psoriasis, selecting an individual afflicted with psoriasis detected with the allele cytosine ("C") of said marker, and administering to said selected individual a therapeutically effective amount of an inhibitor of LFA-1, wherein the individual selected is either heterozygous or homozygous for said allele but preferentially homozygous.

A eleventh embodiment of the fourth aspect of the invention is a method of treating psoriasis comprising detecting the presence or absence of the allele adenine ("A") of the genetic marker SNP_A-2213961 (rs10755496) on one chromosome or both of paired chromosomes in a biological sample of an individual afflicted with psoriasis, selecting an individual afflicted with psoriasis detected with the allele adenine ("A") of said marker, and administering to said selected individual a therapeutically effective amount of an inhibitor of LFA-1, wherein the individual selected is either heterozygous or homozygous for said allele but preferentially homozygous.

A twelfth embodiment of the fourth aspect of the invention is a method of treating psoriasis comprising detecting the presence or absence of the allele cytosine ("C") of the genetic marker SNP_A-2299781 (rs7742750) on one chromosome or both of paired chromosomes in a biological sample of an individual afflicted with psoriasis, selecting an individual afflicted with psoriasis detected with the allele cytosine ("C") of said marker, and administering to said selected individual a therapeutically effective amount of an inhibitor of LFA-1, wherein the individual selected is either heterozygous or homozygous for said allele but preferentially homozygous.

A thirteenth embodiment of the fourth aspect of the invention is the method of treating psoriasis an individual afflicted with psoriasis according to any of the embodiments of the fourth aspect of the invention wherein the individual has not previously been treated with an inhibitor of LFA-1.

A fifth aspect of the invention is an inhibitor of LFA-1 for use in the treatment of psoriasis **characterized in that** the treatment comprises detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or SNP_A-2299781 (rs7742750) in a biological sample of an individual afflicted with psoriasis, selecting an individual afflicted with psoriasis based on the allele of said marker that is detected in the individual, and administering to said selected individual a therapeutically effective amount of an inhibitor of LFA-1. A particularly preferred embodiment of the invention is an inhibitor of LFA-1 for use in the treatment of psoriasis according the fifth aspect of the invention wherein the allele the presence or absence of which is detected is thymine ("T") for the marker SNP_A-2040233 (rs4043263), adenine ("A") for the marker SNP_A-1978609 (rs17501861), cytosine ("C") for the marker SNP_A-1986846 (rs9294506), adenine ("A") for the marker SNP_A-2223123 (rs6485472) or thymine ("T") for the marker SNP_A-2085239 (rs11251116), guanine ("G") for the marker SNP_A-4269573 (rs11037653), guanine ("G") for the marker SNP_A-2010642 (rs4620711), guanine ("G") for the marker SNP_A-4260850 (rs10838166), cytosine ("C") for the marker SNP_A-4239787 (rs4573056), adenine ("A") for the marker SNP_A-2213961 (rs10755496) or cytosine ("C") for the marker SNP_A-2299781 (rs7742750). A further preferred embodiment of the fifth aspect of the invention is an inhibitor of LFA-1 for use in the treatment of psoriasis according to any of the embodiments of the fifth aspect of the invention wherein the individual has not previously been treated with an inhibitor of LFA-1.

A first embodiment of the fifth aspect of the invention is an inhibitor of LFA-1 for use in the treatment of psoriasis **characterized in that** the treatment comprises genotyping of an individual afflicted with psoriasis according the any of the first, second, third, fourth, fifth, sixth, seventh, eighth, ninth, tenth, eleventh embodiment of the first aspect of the invention, selecting an individual afflicted with psoriasis based on the allele of said marker that is detected in the individual, and administering to said selected individual a therapeutically effective amount of an inhibitor of LFA-1.

A sixth aspect of the invention is an inhibitor of LFA-1 for use in the treatment of psoriasis in an individual afflicted with psoriasis **characterized in that** the individual has one allele or any combination of the allele(s) selected from the group consisting of thymine ("T") for the marker SNP_A-2040233 (rs4043263), adenine ("A") for the marker SNP_A-1978609 (rs17501861), cytosine ("C") for the marker SNP_A-1986846 (rs9294506), adenine ("A") for the marker SNP_A-2223123 (rs6485472), thymine ("T") for the marker SNP_A-2085239 (rs11251116), guanine ("G") for the marker SNP_A-4269573 (rs11037653), guanine ("G") for the marker SNP_A-2010642 (rs4620711), guanine ("G") for the marker SNP_A-4260850 (rs10838166), cytosine ("C") for the marker SNP_A-4239787 (rs4573056), adenine ("A") for the marker SNP_A-2213961 (rs10755496) or cytosine ("C") for the marker SNP_A-2299781 (rs7742750).

A first embodiment of the sixth aspect of the invention is an inhibitor of LFA-1 for use in the treatment of psoriasis in an individual afflicted with psoriasis according to the sixth aspect of the invention wherein the individual has not previously been treated with an inhibitor of LFA-1.

A second embodiment of the sixth aspect of the invention is an inhibitor of LFA-1 for use in the treatment of psoriasis in individuals afflicted with psoriasis according to the sixth aspect of the invention wherein at least one of the alleles is present on both paired chromosomes.

A preferred inhibitor of LFA-1 according to any of the embodiments of any of the aspects of the invention is an inhibitor of CD11a. Preferably the inhibitor of LFA-1 or CD11a is a peptide or protein, more preferably an antibody or antigen binding fragment thereof, even more preferred an antibody or antigen binding fragment thereof that binds specifically to CD11 a, and most preferred the inhibitor of LFA-1 is efalizumab, an antigen binding fragment thereof or a derivative thereof comprising all three heavy chain CDRs and/or all three light chain CDRs or at least one CDR of efalizumab.

As used in the specification and the claims, "a" or "an" means one or more unless explicitly stated otherwise. As used herein, "another" means at least a second or more.

An "allele" is a particular form of a gene, genetic marker or other genetic locus, that is distinguishable from other forms of the gene, genetic marker or other genetic locus; e.g. without limitation by its particular nucleotide sequence. The term allele also includes for example without limitation one form of a single nucleotide polymorphism (SNP). An individual can be homozygous for a certain allele in diploid cells; i.e. the allele on both paired chromosomes is identical; or heterozygous for said allele; i.e. the alleles on both paired chromosomes are not identical.

A "genetic marker" or "marker" is an identifiable polymorphic genetic locus. An example without limitation of a genetic marker is a single nucleotide polymorphism (SNP). The SNPs that were analyzed by the present inventors in connection with treatment of psoriasis with efalizumab are those shown in Tables 2 and 3.

A "genotype" as used herein refers to the combination of both alleles of a genetic marker, e.g. without limitation of an SNP, on a single genetic locus on paired (homologous) chromosomes in an individual. "Genotype" as used herein also refers to the combination of alleles of more than one genetic loci, e.g. without limitation of SNPs, on a pair or more than one pair of homologous chromosomes in an individual.

"Genotyping" is a process for determining a genotype of an individual.

The term "inhibitor of LFA-1" or "inhibitor of CD11a" as used herein refers to a compound that binds to and inhibits the activity of LFA-1 or CD11a, respectively, such as the binding of LFA-1 to its ligands, such as ICAM-1, ICAM-2 and ICAM-3. It is understood by the skilled artisan that a compound that blocks the activity of CD11a will also block the activity of LFA-1. "An inhibitor of LFA-1 or CD11a" is for example a peptide or a protein that inhibits the binding of LFA-1 to ICAM-1. Inhibitors of LFA-1 and CD11a are known in the art as well as methods for generating inhibitors of LFA-1 or CD11a (Xingyuan *et al.,* 2006;Anderson and Siahaan, 2003). Examples of such inhibitors are antibodies and antigen binding fragments thereof, or other protein or polypeptide compounds (for example without limitation soluble ICAM-1 and binding fragments and or fusion proteins thereof) comprising more than 10 amino acids that are based on protein scaffolds e.g. from lipocalin ("anticalins"), fibronectin ("adnectins", trinectins), kunitz domains, C-type lectin, transferrin, gamma-crystalline, cysteine-nots, ankyrin repeats ("DARPins") or protein A ("affibodies") as known in the art ((Binz *et al.,* 2004;Gill and Damle, 2006;Mosavi *et al.,* 2004;Nilsson and Tolmachev, 2007;Tomlinson, 2004)). "An inhibitor of LFA-1" or "an inhibitor of CD11a" includes also small molecular weight compounds that inhibit the binding of LFA-1 to ICAM-1, such as for example, organic molecules, other than polypeptides comprising more than 10 amino acids, but including peptides, cyclic peptides comprising not more than 10 amino acids as well as peptidomimetics. Peptidomimetics are compounds that are based on the amino acid sequences found at protein-protein interaction sites and are known in the art ((Sillerud and Larson, 2005)). Efalizumab is an inhibitor of LFA-1. Efalizumab is a humanized antibody that specifically binds to CD11a thereby inhibiting the binding of LFA-1 to ICAM-1.

"Locus" or "genetic locus" refers to a specific location on a chromosome or other genetic material.

"Oligonucleotide" refers to a nucleic acid or a nucleic acid derivative; including without limitation a locked nucleic acid (LNA), peptide nucleic acid (PNA) or bridged nucleic acid (BNA); that is usually between 5 and 100 contiguous bases in length, and most frequently between 5-40, 5-35, 5-30, 5-25, 5-20, 5-15, 5-10, 10-50, 10-40, 10-30, 10-25, 10-20, 15-50, 15-40, 15-30, 15-25, 15-20, 20-50, 20-40, 20-30 or 20-25 contiguous bases in length. The sequence of an oligonucleotide can be designed to specifically hybridize to any of the allelic forms of a genetic marker; such oligonucleotides are referred to as allele-specific probes. If the genetic marker is an SNP, the complementary allele for that SNP can occur at any position within an allele-specific probe. Other oligonucleotides useful in practicing the invention specifically hybridize to a target region adjacent to an SNP with their 3' terminus located one to less than or equal to about 10 nucleotides from the genetic marker locus, preferably ≤ about 5 nucleotides. Such oligonucleotides hybridizing adjacent to an SNP are useful in polymerase-mediated primer extension methods and are referred to herein as "primer-extension oligonucleotides." In a preferred embodiment, the 3'-terminus of a primer-extension oligonucleotide is a deoxynucleotide complementary to the nucleotide located immediately adjacent an SNP.

"Polymorphism" refers of two or more alternate forms (alleles) in a population of a genetic locus that differ in nucleotide sequence or have variable numbers of repeated nucleotide units. Polymorphisms occur in coding regions (exons), non-coding regions of genes or outside of genes. The different alleles of a polymorphism typically occur in a population at different frequencies, with the allele occurring most frequently in a selected population sometimes referenced as the "major" or "wildtype" allele. Diploid organisms may be homozygous or heterozygous for the different alleles that exist. A biallelic polymorphism has two alleles.

"Psoriasis" as used herein refers to all forms of psoriasis, including without limitation plaque psoriasis (*psoriasis vulgaris*), flexural psoriasis (inverse psoriasis), guttate psoriasis, pustular psoriasis, erythrodermic psoriasis, nail psoriasis and psoriatic arthritis.

"Response" to treatment of psoriasis with an inhibitor of LFA-1, an inhibitor of CD11 a or efalizumab, "clinical response" to treatment of psoriasis with an inhibitor of LFA-1, an inhibitor of CD11 a or efalizumab, or the like as used herein is intended to refer to the change in an individual's symptoms of psoriasis following treatment/administration of an inhibitor of LFA-1, an inhibitor of CD11 a or efalizumab, preferably as measured according to the Physician Global Assessment (PGA) score or Psoriasis Area and Severity Index (PASI). The PGA score as used herein is assessed as good or better definition which is defined in the art according the improvement as compared to baseline (day 0) assessed according to Table 1 below after a certain time of treatment, such as for example without limitation after twelve or twenty weeks of treatment.

Table 1: Physician's Global Assessment (PGA) of Change

The global response of all psoriatic lesions to therapy compared with the baseline condition using Study day 0 Body

The physician using the following categories will evaluate diagrams:

| Category | Percentage Improvement | Category Description |
|---|---|---|
| Cleared | 100% | Remission of all clinical signs and symptoms as compared baseline, except for residual manifestations such as mild erythema |
| Excellent | 75%-99% | Improvement of all clinical signs and symptoms as compared with baseline, except for residual manifestations such as mild erythema |
| Good | 50%-74% | Improvement of all clinical signs and symptoms as compared with baseline |
| Fair | 25%-49% | Improvement of all clinical signs and symptoms as compared with baseline |
| Slight | 1%-24% | improvement of all clinical signs and symptoms as compared with baseline |
| Unchanged | | Clinical signs and symptoms unchanged from baseline |
| Worse | | Clinical signs and symptoms deteriorated from baseline |

Alternatively a "response" to treatment of psoriasis with an inhibitor of LFA-1, an inhibitor of CD11 a or efalizumab, "clinical response" to treatment of psoriasis with an inhibitor of LFA-1, an inhibitor of CD11 a or efalizumab can be assessed according to the PASI. The PASI score is known in the art and can be determined by the physician using the questionnaire shown in Fig.1. Based on the questionnaire the PASI score is calculated. The PASI calculated can then be used to determine the improving in % in PASI score according to the formula:

$$\%\ \text{IMPROVEMENT} = \frac{\text{PASI study day 0} - \text{PASI study week 12}}{\text{PASI study day 0}} \times 100$$

An improvement of ≥75% improvement in PASI score after 12 weeks, alternatively up to 20 weeks, of treatment is called PASI75. A patient with PASI75 is considered a treatment responder. An improvement of ≥50% and <75% improvement in PASI score after 12, alternatively up to 20 weeks, of treatment is called PASI50. A patient with PASI50 is considered a partial treatment responder.

"Response rate" is assessed by calculating the number of patient achieving a PGA score of good or better, or PASI50 or 75 after 12 to 20 weeks of treatment.

"Responders" are patients that exhibit a response to treatment of psoriasis with an inhibitor of LFA-1, an inhibitor of CD11 a or efalizumab after 12 to 29 weeks of treatment, preferably responders exhibit a PGA score of good or better or PASI75 or PASI50.

A "single nucleotide polymorphism (SNP)" is a DNA sequence variation occurring when a single nucleotide - A, T, C, or G - in the genome (or other sequence shared between individuals of a species) differs between individuals of a species (or between paired chromosomes in an individual). An SNP is frequently preceded by and followed by highly conserved sequences in the population of interest and thus the location of an SNP is typically made in reference to a consensus nucleic acid sequence of thirty to sixty nucleotides that bracket the genetic marker locus, which is sometimes referred to as a context sequence for the SNP. The skilled artisan understands that the location of a particular SNP may not occur at precisely the same position in a reference or context sequence in each individual in a population of interest due to the presence of one or more insertions or deletions in that individual as compared to the consensus or reference sequence. Moreover, it is routine for the skilled artisan to design robust, specific and accurate assays for detecting the alternative alleles of an SNP in any given individual, when the skilled artisan is provided with the identity of the alternative alleles of the SNP to be detected and one or both of a reference sequence or context sequence of the SNP. Thus, the skilled artisan will understand that specifying the location of an SNP described herein by reference to a particular position in a reference or context sequence (or with respect to an initiation codon in such a sequence) is merely for convenience and that any specifically enumerated nucleotide position literally includes whatever nucleotide position the same SNP is actually located in the same locus in any individual being tested for the presence or absence of a genetic marker of the invention using any of the genotyping methods described herein or other genotyping methods well-known in the art.

The presence or absence of a particular allele or pair of alleles at the locus of a genetic marker of the invention in an individual may be determined by any of a variety of methods well known in the art. Some examples of suitable techniques include, but are not limited to, direct DNA sequencing of the amplified target region, capillary electrophoresis, hybridization of allele-specific probes, single-strand conformation polymorphism analysis, denaturing gradient gel electrophoresis, temperature gradient electrophoresis, mismatch detection; nucleic acid arrays, primer specific extension, protein detection, and other techniques well known in the art (Kim and Misra, 2007;Wang *et al.,* 2007). Such methods are frequently referred to methods for genotyping.

Typically, these methods involve assaying a nucleic acid sample prepared from a biological sample obtained from the individual to determine the identity of a nucleotide or nucleotide pair present of the genetic marker; e.g. without limitation the SNP. Nucleic acid samples may be prepared from virtually any biological sample. For example, convenient samples include whole blood, serum, semen, saliva, tears, fecal matter, urine, sweat, buccal matter, skin and hair. As will be readily appreciated by those skilled in the art, an individual is expected to have two identical alleles for a genetic marker; i.e. be homozygous, or to have two different alleles for a genetic marker; i.e. be heterozygous, for which marker the locus is on an autosomal chromosome, or the locus is on the X chromosome and the individual is a female. Preferred samples contain only somatic cells, and such samples would typically be required when the locus of the genetic marker is on an autosomal or X chromosome. Nucleic acid samples may be prepared for analysis using any technique known to those skilled in the art. Preferably, such techniques result in the production of genomic DNA sufficiently pure for determining the genotype for a desired set of genetic markers on the nucleic acid molecule.

Any amplification technique known to those of skill in the art may be used in practicing the present invention including, but not limited to, polymerase chain reaction (PCR) techniques and other methods (Gingeras *et al.,* 1990). PCR may be carried out using materials and methods known to those of skill in the art. Other suitable amplification methods include the ligase chain reaction (LCR) (Nickerson *et al.,* 2001;Tian *et al.,* 2008), transcription amplification, self-sustained sequence replication; isothermal methods; and nucleic acid-based sequence amplification (NASBA).

The amplified target region is assayed to determine the identity of at least one of the alleles present at a genetic marker locus. If the DNA of both paired chromosomes are represented in the amplified target, it will be readily appreciated by the skilled artisan that only one allele will be detected at a genetic marker locus in individuals who are homozygous for that genetic marker, while two different alleles will be detected if the individual is heterozygous for that genetic marker. The identity of the allele may be identified directly, known as positive-type identification, or by inference, referred to as negative-type identification.

In preferred embodiments, the identity of allele(s) of a genetic marker is determined using a polymerase-mediated primer extension method. Several such methods have been described in the patent and scientific literature and include the "Genetic Bit Analysis" method (WO 92/15712) and the ligase/polymerase mediated genetic bit analysis. (US 5,679,524). Related methods are disclosed in WO 91/02087, WO 90/09455, WO 95/17676, and US 5,302,509 and US 5,945,283. Extended primers containing the complement of the polymorphism may be detected by mass spectrometry as described in United States Patent No. 5605798. Another primer extension method is allele-specific PCR. (Ruano *et al.,* 1994;Ruano and Kidd, 1991;Ruano and Kidd, 1989). In addition, multiple genetic markers may be investigated by simultaneously amplifying multiple regions of the nucleic acid using sets of allele-specific primers as described in WO 89/10414.

Another primer extension method for detecting and analyzing genetic markers such as SNPs employs single-base extension (SBE) of a fluorescently-labeled primer coupled with fluorescence resonance energy transfer (FRET) between the label of the added base and the label of the primer. Typically, the method, such as that described by Chen et al. (Chen *et al.,* 1997), uses a locus-specific oligonucleotide primer labeled on the 5' terminus with 5-carboxyfluorescein (FAM). This labeled primer is designed so that the 3' end is immediately adjacent to the genetic marker locus of interest. The labeled primer is hybridized to the locus, and single base extension of the labeled primer is performed with fluorescently labeled dideoxyribonucleotides (ddNTPs) in dye-terminator sequencing fashion, except that no deoxyribonucleotides are present. An increase in fluorescence of the added ddNTP in response to excitation at the wavelength of the labeled primer is used to infer the identity of the added nucleotide.

In all of the above methods, the accuracy and specificity of an assay designed to detect the identity of the allele (s) of a genetic marker is typically validated by performing the assay on DNA samples in which the identity of the allele (s) at that genetic marker is known. Preferably a sample representing each possible allele is included in the validation process. For diploid loci such as those on autosomal and X chromosomes, the validation samples will typically include a sample that is homozygous for the major allele of the genetic marker, a sample that is homozygous for the minor allele of the genetic marker, and a sample that is heterozygous for the genetic marker. These validation samples are typically also included as controls when performing the assay on a test sample (i.e., a sample in which the identity of the allele (s) of the genetic marker is unknown). The specificity of an assay may also be confirmed by comparing the assay result for a test sample with the result obtained for the same sample using a different type of assay, such as by determining the sequence of an amplified target region believed to contain the genetic marker of interest and comparing the determined sequence to a context sequence based on the reference sequence of the relevant gene. The length of the context sequence necessary to establish that the correct genomic position is being assayed will vary based on the uniqueness of the sequence in the target region (for example, there may be one or more highly homologous sequences located in other genomic regions). The skilled artisan can readily determine an appropriate length for a context sequence for any genetic marker, such as an SNP, using known techniques such as blasting the context sequence against publicly available sequence databases. For amplified target regions, which provide a first level of specificity, examining the context sequence of about 30 to 60 bases on each locus of the genetic marker in known samples is typically sufficient to ensure that the assay design is specific for the genetic marker of interest. Occasionally, a validated assay may fail to provide an unambiguous result for a test sample. This is usually the result of the sample having DNA of insufficient purity or quantity, and an unambiguous result is usually obtained by repurifying or reisolating the DNA sample or by assaying the sample using a different type of assay.

Alternatively, the presence or absence of a genetic marker of the invention may be detected for a genetic marker that is located in a region that codes for a polypeptide and results in an alteration of the polypeptide by detecting, in a protein sample obtained from the individual, the alteration. The polypeptide may be detected for example with out limitation by using a monoclonal antibody specific for that polypeptide and/or the alteration.

An individual afflicted with psoriasis and to be tested and/or treated according to any of the methods and uses described herein is a human subject that is a candidate for treatment with an inhibitor of LFA-1, an inhibitor of CD11a or an efalizumab. In a preferred embodiment, the individual has been diagnosed with psoriasis, or exhibits a symptom of psoriasis, a disease for which the inhibitor of LFA-1, the inhibitor of CD11a or efalizumab is approved.

Efalizumab is currently not approved for treating diseases other than psoriasis. Nevertheless, other diseases have been successfully treated with efalizumab, and comprise for example without limitation rejection of a transplant; e.g. without limitation kidney, heart, liver, lung, cornea; aphthous stomatitis; atopic dermatitis; alopecia area; lichen planus; dermatomyositis; granuloma annulare; hidradenitis suppurativa; pityriasis rubra pilaris; pyoderma gangrenosum and discoid and cutaneous lupus erythematosus. In other embodiments of the invention an individual to be tested and/or treated according to any of the methods described herein is a human subject that has been diagnosed with psoriasis, or exhibits a symptom of rejection of a transplant; e.g. without limitation kidney, heart, liver, lung, cornea; aphthous stomatitis; atopic dermatitis; alopecia area; lichen planus; dermatomyositis; granuloma annulare; hidradenitis suppurativa; pityriasis rubra pilaris; pyoderma gangrenosum and discoid and cutaneous lupus erythematosus. Consequently, the skilled artisan understands that the genetic markers of the invention are also useful to predict the clinical response of treatment of any of the above diseases with efalizumab. The skilled artisan also understand the methods and uses of the invention can also be applied to individuals that are afflicted with any of the above diseases.

The diagnostic methods and kits of the invention are useful in clinical diagnostic applications. However, as used herein, the term "diagnostic" is not limited to clinical or medical uses, and the diagnostic methods and kits of the invention claimed herein are also useful in any research application in which it is desirable to test a subject for the presence or absence of any genetic marker described hereinabove.

In some embodiments, the oligonucleotides in the kit are either allele-specific probes or allele-specific primers. In other embodiments, the kit comprises primer-extension oligonucleotides. In still further embodiments, the set of oligonucleotides is a combination of allele-specific probes, allele-specific primers, or primer-extension oligonucleotides.

The composition and length of each oligonucleotide in a kit of the invention will depend on the nature of the genomic region containing the genetic marker of the invention as well as the type of assay to be performed with the oligonucleotide and is readily determined by the skilled artisan. For example, the polynucleotide to be used in the assay may constitute an amplification product, and thus the required specificity of the oligonucleotide is with respect to hybridization to the target region in the amplification product rather than in genomic DNA isolated from the individual.

In preferred embodiments, each oligonucleotide in the kit is a perfect complement of its target region. An oligonucleotide is said to be a "perfect" or "complete" complement of another nucleic acid molecule if every nucleotide of one of the molecules is complementary to the nucleotide at the corresponding position of the other molecule. While perfectly complementary oligonucleotides are preferred for detecting polymorphisms, departures from complete complementarity are contemplated where such departures do not prevent the molecule from specifically hybridizing to the target region as defined above. For example, an oligonucleotide primer may have a non-complementary fragment at its 5' end, with the remainder of the primer being completely complementary to the target region. Alternatively, non-complementary nucleotides may be interspersed into the probe or primer as long as the resulting probe or primer is still capable of specifically hybridizing to the target region.

In some preferred embodiments, each oligonucleotide in the kit specifically hybridizes to its target region under stringent hybridization conditions. Stringent hybridization conditions are sequence-dependent and vary depending on the circumstances. Generally, stringent conditions are selected to be about 5°C lower than the thermal melting point ($T_m$) for the specific sequence at a defined ionic strength and pH. The $T_m$ is the temperature (under defined ionic strength, pH, and nucleic acid concentration) at which 50% of the probes complementary to the target sequence hybridize to the target sequence at equilibrium. As the target sequences are generally present in excess, at $T_m$, 50% of the probes are occupied at equilibrium. Typically, stringent conditions include a salt concentration of at least about 0.01 to 1.0 M Na ion concentration (or other salts) at pH 7.0 to 8.3 and the temperature is at least about 25°C for short oligonucleotide probes (e.g., 10 to 50 nucleotides). Stringent conditions can also be achieved with the addition of destabilizing agents such as formamide. For example, conditions of 5 x SSPE (750 mM NaCl, 50 mM NaPhosphate, 5 mM EDTA, pH 7.4) and a temperature of 25-30°C are suitable for allele-specific probe hybridizations.

A preferred, non-limiting example of stringent hybridization conditions includes hybridization in 4 x sodium chloride/sodium citrate (SSC), at about 65-70°C (or alternatively hybridization in 4 x SSC plus 50% formamide at about 42-50°C) followed by one or more washes in 1 x SSC, at about 65-70°C. A preferred, non-limiting example of highly stringent hybridization conditions includes hybridization in 1 x SSC, at about 65-70°C (or alternatively hybridization in 1 x SSC plus 50% formamide at about 42-50°C) followed by one or more washes in 0.3 x SSC, at about 65-70°C. A preferred, non-limiting example of reduced stringency hybridization conditions includes hybridization in 4 x SSC, at about 50-60°C (or alternatively hybridization in 6 x SSC plus 50% formamide at about 40-45°C) followed by one or more washes in 2 x SSC, at about 50-60°C. Ranges intermediate to the above-recited values, e.g., at 65-70°C or at 42-50°C are also intended to be encompassed by the present invention. SSPE (1 x SSPE is 0.15 M NaCl, 10 mM $NaH_2PO_4$, and 1.25 mM EDTA, pH 7.4) can be substituted for SSC (1 x SSC is 0.15 M NaCl and 15 mM sodium citrate) in the hybridization and wash buffers; washes are performed for 15 minutes each after hybridization is complete.

The hybridization temperature for hybrids anticipated to be less than 50 base pairs in length should be 5-10°C less than the melting temperature ($T_m$) of the hybrid, where $T_m$ is determined according to the following equations. For hybrids less than 18 base pairs in length, $T_m$ (°C) = 2(# of A + T bases) + 4(# of G + C bases). For hybrids between 18 and 49 base pairs in length, $T_m$ (°C) = 81.5 + 16.6(log10[$Na^+$]) + 0.41(%G+C) - (600/N), where N is the number of bases in the hybrid, and [$Na^+$] is the concentration of sodium ions in the hybridization buffer ([$Na^+$] for 1 x SSC = 0.165 M).

The oligonucleotides in kits of the invention may be comprised of any phosphorylation state of ribonucleotides, deoxyribonucleotides, and acyclic nucleotide derivatives, and other functionally equivalent derivatives. Alternatively, the oligonucleotides may have a phosphate-free backbone, which may be comprised of linkages such as carboxymethyl, acetamidate, carbamate, polyamide [peptide nucleic acid (PNA)] and the like. The oligonucleotides may be prepared by chemical synthesis using any suitable methodology known in the art, or may be derived from a biological sample, for example, by restriction digestion. The oligonucleotides may contain a detectable label, according to any technique known in the art, including use of radiolabels, fluorescent labels, enzymatic labels, proteins, haptens, antibodies, sequence tags and the like. The oligonucleotides in the kit may be manufactured and marketed as analyte specific reagents (ASRs) or may be constitute components of an approved diagnostic device.

In other preferred embodiments, the kit includes an instruction manual that describes the various ways the kit may be used to detect the presence or absence of a genetic marker of the invention.

In a preferred embodiment, the set of oligonucleotides in the kit are allele-specific oligonucleotides. As used herein, the term allele-specific oligonucleotide (ASO) means an oligonucleotide that is able, under sufficiently stringent conditions, to hybridize specifically to one allele of a genetic marker, at a target region containing the genetic marker while not hybridizing to the same region containing a different allele. As understood by the skilled artisan, allele-specificity will depend upon a variety of readily optimized stringency conditions, including salt and formamide concentrations, as well as temperatures for both the hybridization and washing steps.

Typically, an ASO will be perfectly complementary to one allele while containing a single mismatch for another allele. In ASO probes, the single mismatch is preferably within a central position of the oligonucleotide probe as it aligns with the genetic marker in the target region (e.g., approximately the 7th or 8th position in a 15mer, the 8th or 9th position in a 16mer, and the 10th or 11th position in a 20mer). The single mismatch in ASO primers is located at the 3' terminal nucleotide, or preferably at the 3' penultimate nucleotide. ASO probes and primers hybridizing to either the coding or non-coding strand are contemplated by the invention.

In other preferred embodiments, the kit comprises a pair of allele-specific oligonucleotides for a genetic marker of the invention to be assayed, with one member of the pair being specific for one allele and the other member being specific for another allele. In such embodiments, the oligonucleotides in the pair may have different lengths or have different detectable labels to allow the user of the kit to determine which allele-specific oligonucleotide has specifically hybridized to the target region, and thus determine which allele is present in the individual at the assayed marker locus.

In still other preferred embodiments, the oligonucleotides in the kit are primer-extension oligonucleotides. Termination mixes for polymerase-mediated extension from any of these oligonucleotides are chosen to terminate extension of the oligonucleotide at the genetic marker of interest, or one base thereafter, depending on the alternative nucleotides present at the marker locus.

Treatment of psoriasis with efalizumab is performed according to the prescription information and/or as known in the art. In general, the treatment of psoriasis will involve an initial subcutaneous injection of 0.7 mg/kg efalizumab followed by a weekly subcutaneous injection of 1 mg/kg efalizumab. The treatment course may be applied for an initial period of 12 weeks or shorter or may be continuous. The skilled artisan is aware of modifications of the treatment regimen and/or period that may be required or prudent depending on the symptoms and/or physical status of the individual afflicted with psoriasis and/or on the clinical response to the treatment.

Diseases other than psoriasis that may be treated with efalizumab depending on the symptoms or physical status of the individual afflicted are for example without limitation rejection of a transplant; e.g. without limitation kidney, heart, liver, lung, cornea; aphthous stomatitis; atopic dermatitis; alopecia area; lichen planus; dermatomyositis; granuloma annulare; hidradenitis suppurativa; pityriasis rubra pilaris; pyoderma gangrenosum and discoid and cutaneous lupus erythematosus. The skilled artisan is aware of treatment regimen and/or the period that may be required or prudent depending on the symptoms and/or physical status of the individual afflicted with the disease and/or on the clinical response to the treatment.

Other embodiments of the invention within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims that follow the examples.

## EXAMPLES

*Example* 1: *Identification and selection* of *genetic markers associated with the clinical response of treatment of psoriasis with efalizumab*

An exploratory analysis, non-hypothesis driven, hypothesis generating, whole genome scan genotyping using 500K Affymetrix DNA chip was carried out using blood samples from psoriatic patients treated with efalizumab during 12 or 20 weeks.

The following criteria were used to assess the association between genetic variants and clinical parameters: p-value, odd ratio, relative risk, minor allelic frequency, missing data. The clinical parameters used were: PGA, PASI50, PASI75, at baseline and after 12 and 20 weeks of treatments. Non responders and responders were defined based on clinical parameters according to the PGA or PASI score as known in the art and disclosed herein. Selection of associated markers was driven by the statistical results classified based on the observed p-value (<0.05) after correction for multiple testing (Bonferroni and False Discovery Rate-FDR) and filtering for minor allelic frequency (<5%) and missing data (>5%).

***Genotyping of individuals afflicted with psoriasis for genetic markers*** *SAMPLE PRE-PROCESSING*

Blood samples for DNA extraction were collected in EDTA tubes and stored at -70 °C until processing.

DNA was extracted from whole blood using QIAgen kit. After extraction, DNA samples absorbance was measured at wavelengths of 260 nm and 280 nm using a spectrophotometer and gel electrophoresis on agarose gels were performed to estimate the quantity and quality of DNA. 260 nm/280 nm absorbance ratio and DNA concentration was calculated from the 280 nm absorbance measure using a spectrophotometer correspond to the first quality control (QC. 1). Electrophoresis on agarose gel corresponds to the second quality control (QC.2). All DNA samples passed the acceptance criteria defined for QC.1 (absorbance ratio was between 1.7 and 2.0 and DNA concentration was above 50 ng/μL). All DNA samples passed the acceptance criteria defined for QC.2 (for each sample, one band was visible on agarose gel after electrophoresis at a high molecular weight corresponding to non-degraded genomic DNA). An aliquot from each sample was distributed into micro plates (96 wells). Each micro-plate contained also a negative control and a reference genomic DNA called "103". The micro-plates were transferred for genotyping (Affymetrix SNP genotyping).

***Affymetrix Genotyping***

The genotyping was performed to screen the whole genome (process called Whole Genome Scan). There was no hypothesis concerning any of the variants (Single Nucleotide Polymorphisms or SNPs). The SNPs were randomly distributed in all the chromosomes and were used as tagging markers of the corresponding genomic area. Affymetrix Mapping 500K Assays were used to perform the Whole Genome Scan. The Affymetrix technology is based on DNA chips allowing the genotyping of approximately 500,000 SNPs per patient (Docherty *et al.,* 2007). Five hundred and forty three samples and 2 commercial control samples had been processed. For each plate contacting the samples, genomic DNA samples were digested with Nsp I and Sty I restriction endonucleases, ligated with specific adaptors for Nsp or Sty. The product of ligation was amplified by Polymerase Chain Reactions (PCR) in triplicate to produce a large amount. The three PCR products were pooled, purified, quantified, fragmented and labelled.

PCR amplification step and fragmentation step were evaluated using electrophoresis agarose gel corresponding respectively to quality control 3 (QC.3) and quality control 5 (QC.5). Quantification step was measured using spectrophotometer corresponding to quality control 4 (QC.4). QC.3: Amplification by PCR: All samples were amplified in the three products according to the acceptance criteria (size range was included between 200 and 1100 base pairs (bp)).

QC.4: Quantification of the purified PCR product: All micro-plates passed the acceptance criteria, DNA quantity above 90 $\mu$ g (more or less 20%) for at least 80% of the samples on a micro-plate.

QC.5: PCR product fragmentation: For at least 80% of the samples on a micro-plate, the size of fragmentation products were above 180 bp and matched the acceptance criteria.

The 1100 labelled products (543 samples and the 7 reference genomic DNA 103 controls in the two enzyme process) were hybridized onto Affymetrix GeneChips Mapping 500K as known in the art.

After hybridization and staining the Affymetrix GeneChips (or arrays) were scanned: the image generated (DAT files) were automatically analyzed by Affymetrix GeneChip Operating Software (GCOS) which computes a single intensity value for each probe cell on an array (CEL files). The CEL files are analyzed using the Affymetrix GeneChip Genotyping Analysis Software (GTYPE) containing three algorithms: Mapping Algorithm (MPAM), Dynamic Model Model Algorithm (DM) and Mapping Tool Algorithm (Kennedy *et al.,* 2003;Di *et al.,* 2005;Matsuzaki *et al.,* 2004). GTYPE algorithms are used to evaluate the quality of the fluorescent signal (cell intensity data). Each SNP that can be detected with the array has a group of probes cells, known as a probe set, on the array. For each array GTYPE algorithms determine the alleles of the SNPs represented in the sample. GTPYE generates a CHP file by experiment (containing the genotype calls) and a report file. The report file contains the Call Rate (% of positive calls for all probe sets) and genotype call for 50 probe sets common across the two Nsp and Sty arrays (used for discordant evaluation in the GTYPE quality control).

One thousand and eighty six GeneChips have been run for the 543 samples (two arrays per sample) in several batches (between 36 to 64 arrays per batch).

When the sample Call Rate (CR) was below 93%, the reaction mix of the same sample was hybridized onto a new GeneChip and analyzed a second time. Seven hundred and ninety six GeneChips had a CR above 93% and 290 had a CR below 93%. The remaining 290 fragmentation products were hybridized on a second GeneChip. After the second hybridization run, 161 GeneChips had a CR above 93%. One hundred and twenty nine GeneChips had a CR below 93%, the corresponding samples were selected for a second run. After the first run (hybridization and re-hybridization), 957 (796 + 161) GeneChips had a CR above 93%.

The discordance call (homozygote allele 1/allele 1 versus homozygote allele 2/allele 2 (also called hard discordant) and homozygote allele 1/allele 1 versus heterozygote allele 1/allele 2 (aslo called soft discordant) was evaluated by comparison of the genotypes for 50 probe sets common to the Nsp and Sty arrays.

For each sample, the acceptance criteria of the GTYPE quality control step (QC.6) were set above a CR of 93% for both GeneChips (Nsp and Sty) with a number of soft discordant below 1 % and no hard discordant. When soft discordant was above 1% or when a hard discordant was detected, samples were re-run from the genomic DNA aliquot for both Nsp and Sty enzyme process. When the CR, for at least one enzyme, was below 93%, the sample was also re-run from the genomic DNA aliquot through the corresponding enzyme process.

Eleven samples did not pass the allelic call discordance acceptance criteria of QC.6. One hundred and forty samples were re-processed (named Redo). During the redo process all samples passed the acceptance criteria set for QC.3, QC.4 and QC.5 steps:

QC.3: all samples were amplified and the size ranges of PCR product were between 200 and 1100 pb.

QC.4: all DNA quantities of purified PCR product were above 90 g.

QC.5: all fragmented PCR product were below 180 pb. During the Redo process, 100 GeneChips had a CR above 93% at the first hybridization. Forty GeneChips had a CR below 93% and were re-hybridized. In second hybridization, twenty-four had a CR above 93%. Sixteen did not reach the acceptance criteria.

Discordance was re-evaluated for the 543 samples. 12 samples had more than 1% of soft discordant and no hard discordant was detected for the 50 SNP common to the two arrays. 23 patients were removed based on QC.6 acceptance criteria after the redo process (some samples have both CR above 93% for two GeneChips and discordance below 1 %).

BRLMM (Bayesian Robust Linear Model with Mahalanobis Distance Classifier) is a model based algorithm which needs to be run on multiple CEL files at once to estimate probe effect and SNP cluster parameters (world wide web affymetrix.com/support/technical/whitepapers/brlmm_whitepaper.pdf, (Rabbee and Speed, 2006;Lin *et al.,* 2008). BRLMM perform a multiple-chip analysis fitting probe effects to increase precision on signal estimates for two alleles of each SNP, followed by a Bayesian classification approach to make genotype calls.

CEL files were split in 2 x 4 batches (520 Nsp I and 520 Sty I CEL files) for the analysis with BRLMM algorithm.

From brlmm.report files (containing gender and call rate) and brlmm.calls files, discordance between homozygote allele 1/ allele 1-homozygote allele 2/allele2 (hard discordant) and homozygote allele 1/allele 1-heterozygote allele 1/allele 2 (soft discordant) was evaluated for the 50 probe sets shared between the two Nsp and Sty arrays, for each sample. The acceptance criteria of BRLMM quality control step (QC.7) was set below 1% of soft discordant and no hard discordant. Two patients did not pass the acceptance criteria of QC.7 and were removed from the subsequent analysis. After all quality control steps, twenty-five samples were removed from the subsequent analyses (twenty-three after QC. 6 and two after BRLMM QC.7). The corresponding CEL files of the 518 DNA samples selected after the genotyping process containing the genotyping call was used for further processing.

***Statistics***

Association of SNPs with the phenotypic traits considered was assessed using a p-value calculated with an exact Fisher tests (testing the null hypothesis that differences in allele distribution between the two phenotypic groups are due only to sampling fluctuation, the two groups being drawn from the same population).

SNPs that did not meet criteria of minimal call rate (missing data proportion < 5%) and minimal frequency of polymorphism (minor allele frequency > 5%) were filtered out prior to this analysis.

Computation of p-values as well as odds ratio, relative risks and their confidence intervals at 95%, were performed using the FREQ procedure from the SAS software (release 9.1.3, service pack 4).

Input data set for a given pair of phenotypic groups was provided as genotypic contingency tables. Automatic assignment of the analytical process to each data set was performed using a SAS program which implements sequentially SNP filtering, set up of allelic contingency tables in a format suitable for FREQ input, execution of the FREQ procedure, formatting of its output and addition of several derived association metrics for each SNP (Bonferroni-corrected p-values , Benjamini-Hochberg estimated FDR upper bound, true positive rates and false-positive rates of minor and major alleles as predictors of phenotype).

The selection criteria of "best" predictive markers were based Bonferroni-corrected p-values, with a threshold of 0.05 and/or Benjamini-Hochberg estimated FDR upper bound of 10 %.

***Analysis of association based on PGA response***

Two SNPs qualify as associated to response as assessed by the PGA score, based on the selection criteria: SNP_A-2040233 and SNP_A-1978609.

Fig. 2b provides a summary of the allelic Odd Ratio (OR) and Relative Risk (RR) from the Whole Genome Scan results output for SNP_A-2040233. The C allele shows an OR = 2.26 (95%Cl: 1.66 - 3.08). The RR for both alleles (RR_min and RR_maj) are also displayed. Fig. 2a shows the distribution of marker SNP_A-2040233 in the two categories of response.

The SNP is located on chromosome 17, close to metallo-peptidase domain 11 (ADAM11) gene, a gene involved in cell adhesion and trafficking.

Fig. 3 provides the corresponding information for marker a SNP_A-1978609.

This marker is located on chromosome 4, not in or close to a known gene.

***Analysis of association based on PASI50***

Four SNPs qualify as associated to response according to PASI50 based on the selection criteria: SNP_A-1986846, SNP_A-4239787 (rs4573056), SNP_A-2213961 (rs10755496) and SNP_A-2299781 (rs7742750). All these SNPs are located on chromosome 6 and are in linkage disequilibrium.

Fig. 4b provides a summary of the allelic OR and RR from the WGS results output for SNP_A-1986846. The RR for both alleles are also displayed (RR_min and RR_maj).

For the SNP with the "best" p-value: SNP_A-1986846, the G allele shows an OR = 2.52 (95%Cl: 1.72 - 3.70). Fig. 4a shows the distribution of the SNP in the different response groups based on information on allelic and genotypic counts.

***Analysis of association based on PASI75***

Five SNPs qualify as associated to response according to PASI75 based on the selection criteria: SNP_A-2223123, SNP_A-2085239, SNP_A-4269573 (rs11037653), and SNP_A-2299781 (rs7742750), SNP_A-2010642 (rs4620711) and SNP_A-4260850 (rs10838166). All these SNPs are located on chromosome 11, except SNP_A-2085239, located on chromosome 10, in a region without a known gene.

Fig. 5b provides a summary of the allelic OR and RR from the WGS results output for SNP_A-2223123. The C allele shows an OR = 0.5 (95%Cl: 0.38 - 0.65). The RR for both alleles are also displayed (RR_min and RRmaj).

Fig. 5a shows the distribution of SNP with the smallest p value located on chromosome 11, SNP_A-2223123. It contains information on allelic and genotypic counts in the two PASI75 defined response groups. SNP_A-2223123 and SNP_A-4269573 (rs11037653), SNP_A-2010642 (rs4620711) and SNP_A-4260850 (rs10838166) are located within the HydroxySteroid (17-beta) Dehydrogenase 12 (HSD17B12) gene locus.

Fig. 6 shows the corresponding information on the distribution of the SNP located on chromosome 10, SNP_A-2085239. It contains information on allelic and genotypic counts in the PASI75 defined response groups.

All response markers identified during this analysis were risk markers (i.e. no marker was 100% associated with the clinical outcome), with relative risks of 1.4-2.0 and fairly narrow 95% confidence intervals. The best response rates observed were approximately 95% of responders (based on PGA response) that represent 13% of the PGx population (marker SNP_A-1978609), and approximately 80% of responders (based on PASI75) that represent 4% of the PGx population (marker SNP_A-2085239). The worst response rates observed were approximately 65% of responders (based on PGA response) that represent 15% of the PGx population (marker SNP_A-2040233) and approximately 25% of responders (based on PASI 75) that represent 13% of the PGx population (marker SNP_A-2223123).

In addition, it was noted that two markers identified are located in or close to the functional structure of two genes. Marker SNP_A-2040233 is close to the gene ADAM11 also called metallo-peptidase domain 11 that is involved in cell adhesion and trafficking. Marker SNP_A-2223123 is in the gene HSD17B12 also called Hydroxysteroid (17-beta) Dehydrogenase 12 that is involved in steroid metabolism. By their functions these two genes maybe closely related to the psoriasis physio-pathology and efalizumab mechanism of action.

Table 2: Genetic markers

| Marker | Synonym | Allele 1 | Allele 2 | Genomic locus | Flanking sequences |
|---|---|---|---|---|---|
| SNP_A-2040233 | rs4043263 | C (forward) | T (forward) | ADAM11 | TACCCACGCA GGGGACACTT AGGGCTCCAG GGTTTGGTCC GAGGCTGAGA GGCATCTGGT<br>GACTCTGCAC TGACCATTTT TTTTTTTTCT TGAGACAGAG CCTTGCTCTG TCGCCAAGGC<br>TGGAGTGCAG TGGTGTGACC TCAGCTCACT GCAACCTCTG CCTCCCGGGT TCAAGCAATT<br>CTCTCTCTCA GCCTCCCAAG TAGTTGGGAC TGCAGGCAAG CGCCACCACA CCTGGCTAAT<br>TTTTGTATCT TTAGTAGAGA CAGGGTTTCA CCATGTTGGC CAGGCTGGTC TCGAACTCCT<br>GACTTCAAGT GATCCACCTG CCTTGGCCTC CCAAGTGTTG GGATTACAGG CGTGAGCCAC<br>CACGCTCGGC CTACACTGAC CTTTGAGAGT CACAAAGGCT GAGCCTCACC TGTGCCCTCT<br>*CTGCCTTCCT GCCATTCATC TGCCAGAAGC CACCCTTTCC CTATCTGGAG GGTCAGCTGA*<br>ACCACACATC AGACC<br>**Y**<br>GGATAGACTG GGAACTGGGA ACTGAGAAAG GGTTAAGCAT GGGCATGGAG GAGTTGAGAG<br>GCAGAAGCAA AAAGAAAGTT AAAAATAATG TTATTCAGCC TAGAGCAAGG AAGACAAGGG<br>CACAGCTAGA CTTCAGTTTG TGACTGATCG GTTTGAGAAT GATTCTTGCT TTTTTCCCCC<br>TGCACTCTGA ATAGGAAGAA GCTGACCTTC TGCTGCAGGA GAGACTGGAG TTAGATGTTA<br>CCCAGTGTCC TGCGGCGACC AGATGCCAGA GTAGGAATCT AAGCTGAACG TGGCCCTGGG<br>TCCTACTCTT TCGGCATGTG ATCCGAGGTG GCCTTGGGAA CCCTACCTGT TCAGGAATGG<br>GGGGCTATGA TCCCAAAGCT TCTAGCTGCC AATT |
| SNP_A-1978609 | rs17501861 | A (forward) | T (forward) | | TTATACATAT ATATACAGTA TACAGTATAT ATACATATAC TGTATATATG TATATTATAT<br>GTACATTGTA TATATATAAT GTATACAGTA TACTATATAT ACACACACAC ACACACACAC<br>ACACATAAAT ACAAGACTAT GGAGACCTAA CATGTTCCCT GTGTTTGCCC AGCATTTGCT<br>TTCGCTTTTA TTAAAATCTG ATAGTTTACC CTTTGCCTGG ATTTCCATGA GTTATGAGTA<br>TGTCCTTCCA ATAGTACTTA CCACAAATAT AACTTGATTG AAAGTAGTTT AAGTTGCTCT<br>**W**<br>CAAAGGTTAT TTCTAAAATA CTGTGTTTCT CATTGAGATT TCTCTTTGAG ATATTACCTA<br>ACTTGATCTA TATATTAGGT AGATATACAA TATATTAGGT AGATATACAA TATCTATATA<br>TTAGGTAGAT ATACAATATA TTCCAATATG ACATGCACAG TACATAAGAT AAAGCCCTCA<br>CACTTTCACA CCACTAAATA TATTTAAACA ACGTCAAATT TTGAAAGAAA TTTCAAAATT<br>CAGGACCAAA TAGTCTGATA AAGACTTACA ATAAATATAT TGATTTTTAT TATAATGATT |
| SNP_A-1986846 | rs9294506 | C (forward) | G (forward) | | TATAACATCT AGGAGTATTG TTTCATAAAA ATAGTATGTA AATATATAAC TGAATTACTA<br>CATAATGAGG AAATTCCCAT GGGCAAAAAA TTACCAGGAT TATGTATTCT GATACATGAG<br>CACAGGAATC TGCCTGCTTG GGCCATCTGC CTACACCATG TGGTCCACAG CACTGCCTTT<br>AAAGGGCTAT ATGTGTAGGG GACATAAGAC AATGTCTGAA GCCTGCACAA AGCCAGAACC<br>*TCTGAAAGGA GACACATGGA ATGAAACAGG GATCTAGACA AACAGCCCTA CCTCGCAGAA*<br>**S** |

(continued)

| Marker | Synonym | Allele 1 | Allele 2 | Genomic locus | Flanking sequences |
|---|---|---|---|---|---|
| | | | | | GAATTTTTTA AACTATTAGT AATAGCAATA ACTTTTTTTT GAAAAAAAAA ATACGCATCT<br>ACAGTCGATT GCTCTGGACA TTGATTCTGG GTGAAAGGGA AGATCTCCCC TGATAATTCA<br>GCACGTGATT GGTCCTCATT TGGGTTTGAG GCTAATATTC ACATTCTCTT TATGGCTTGA<br>ATGATACCAA GTCAATCTTT AAGAGGTTGA ACATGTTGGG AGTGCTTTCT GAGAAAATCT<br>TAGGCAAATA TCCTCTTGAA GAAAATATCT CAAATAATTA CCAAAATATA AACAAGAAAA |
| SNP_A-2223123 | rs6485472 | C (reverse) | A (reverse) | HSD17B12 | CAGGTCTCTC TTCCCACCTG CACTGTGGGA CAGTGATGAG GTTTCTAAGG AAGGTAGAAA<br>ACAGTGACTT ACTTCAAAGG AGACCTATTA CGTCCTCTAT GACAGTAGAA TTTTTATAGA<br>GGTGTGGACA AGGATACCAC AACAGTCTGA TAGAATATTA AAGCACTTAT AAAATAAAGG<br>CTTGGCAATT TTTTTTTAAT CCAGGTGACA ATTAAGGGAG TCTTATTGGG AATTTCACAG<br>GTCTGAGGGA ATTCCATGAG AGAGCATATC TATAGGAAGC TGTAAGTGTG AGGGACTGAG<br>**K**<br>TTTATAGAGT TGGGCGAATT TAATGTCTGG CTTGTGGGAG GACTAGTAAT TAAAGAGAAT<br>ATGTTTATAT GAAAAGGTAC TTTCCTGTCA GCAATTGGTG TTTTCTGCTT TGGGTTTTTT<br>CGCAACATTT TAGTTCTAGT AGATCCTCAG TAATTTGTAC CACTGAGACA GCATTGTGTG<br>GCTGTTAGCA AGTAAGCAGT ATAATGAATT AAGAATGTTG CATTACAGAA AACATTGCTT<br>GACTTGTTTA TTTGGATATT TGTCATAACA AACTAGAAAA TGCGCTGAAG TATGAGCAAC |

(continued)

| Marker | Synonym | Allele 1 | Allele 2 | Genomic locus | Flanking sequences |
|---|---|---|---|---|---|
| SNP_A-2085239 | rs11251116 | T (reverse) | G (reverse) | | TGCCTAGAGG TCTCCTGTCC AGTAAAGACT AGATGAAGAA AGTAAGAACT GAAAAGGGAT AAAATAATGA ACTTTATGTT TTCTACTTGT TTTTCTGTTT GTTTAGGTAT GGTGTCTTGC TCTGCTGCCC AAGCTGGAGT TCAGTGGTGC AATCATAGCT ATGTATATTA CCTTTTCTTT AAAAAAGTTT AACTTTTAGA ACAGTTTTCA ATTCCCAGCA AAATTGAGAA AAAGGTACAG GAAGTTCTCA TATATTCCCC AGCTCCCACA CATGGACATG CTCCCGCCAT CAGCACCTCC CCTGGAGGGT GCATTTGTTA CAATGGATGA ACCTACATGG ACATATCACT GTTACATAGA GTCCACAGTG CAACTGAGGG CTCGCTCTGG GTATTGTACA CTTTGTGGGT TTGAACAGAT GTATAATGAC ATGGAGTCAC CATTGCAGCA TCATAGAGTA GTTTCACCAG CCCCCAGATC CCCCAAATCC GCTGTGCTCA GCTTGCTCAT CCCTGGCTTC CCCTAACCCA GGGCGACCAC TGGTCTTTTT ACTACCTTTA TAGTTTTGCC ATTTCCAAAA TGTTGCTGAG TTGAATGACA CAGTATATAG CCTTTTCAGA TTGGCTTCTT TAACCTGGCA ATATGCATTG AATGTTACTC CATGTTTTTG CATGATGATA GTTCACTTCC TTTTTTCAGC AGTGTATAAT ATTCCATCAT CTGGATGTAC CGCCGTTTAT TTATCCATGT CACTGAAGGA CATCTTGGTT GCTCCCAAGT TTTGGAAGTT ATGAGTAAGG CTGCTATAAA CATCTGTGGA TAGGTTTTTA TCTGGACATA TTTTAACGCT GATAAATACC AAGGAGTGTG ATTACTGGAT AGTATGGTAA GCATATGTTT AATTGGGTAG GAAATTGCAA GCTGTCTCCA CAGTGGCTGT ACCATTTTGC AATTCCACCA GTAAAAGATG AGAGTCCCTG TTGCTCCACA TCCTCTCCAG CATGTTGGTG TTGTCAGTGG GTTT<br>**M**<br>GGCCATTCCA CTGAGTGTGC AGTCGAGTCT TATTGTTTTA ACTTCCATTT CCCTGAGGAC CTATGACGTG GAGCATCTTC TCCTATGCGT GTTTGCCAGC TGTAGATGTT CTTTGATGAG GTGTCTGTTC AGGTCCTTGG CGCATTTTTT AATCCGGTTG TTTGTTTTCT TATTGTTGAG TTTTAGGACT TCTTTGTATA TATTTTGGAT AACAGTCCTT TATCAGATGT GTATTTTGCA |
| | | | | | AATATATCCT CCCAGTGAGT GGCTTGTCTT GGCAGTGTTT TTCACAGAGC AGAAGTTGTT AATTTGTTTG AAGTTCAGCT AATCAGTCCT TTCACGGACT GTGCCTTTGG TGTTTTATCT AAAACATCAT GGTTGTGCCC AAGGTGGCCA AGATTTTCCC CTCTGATATC TTCCAGTTTA GAGTTTGCCT TTTGTGGTTA TGTCTAGGAT CCATTTTGAG TAAAGTTTTG TGATGGGTGT AAGGTCTGTG TCTAGATCCT GCCCCCCCCC ACACACTTTC TTTGCCTGTG GATGTCTGGT TGTTCCAGCA CCATTTGTTG AAAAATGATC TTTTCTCTAC TGAGTTGCCT TTGCCCCTTT GTCAAAGATG GGTTGACTGC ATCTGGGTGG GTCTGTTTCT GAGCTCTGTG TTCTGTTCCC TTGATCCATT TGTTCTTTCT TTCACTAATG TCACACTGTA TTGATTACCA TGGTTGGCAG TAACTCTTCA ATGTTTACTA CTTTTAATAC ACTAAATACA GTTCCAAGAC TATGGGTTTT TAAAGCTCTA AAATATCTTT TATTCTAAAT AGTTTAAACT GTATCCTTTT TG |

(continued)

| Marker | Synonym | Allele 1 | Allele 2 | Genomic locus | Flanking sequences |
|---|---|---|---|---|---|
| SNP_A-4269573 | rs11037653 | A (forward) | G (forward) | HSD17B12 | TAAGAAAAAT AAATCACCAG GGTACACCCC ATTTGTTTGA AGTGTTAAGC ACCAAACAGT<br>CAGTGCTTAA TAAAGAAAAT CAATATTTTC AGAATTACAA AGGAAAATGC TAAAGCCCTT<br>AGTCATGAAA GAAACATTAA GTTTTCAGTA AGATAAGTGG AAAGGAAACA GCCATGAATT<br>GTGAGAATTT ATCCTGAAAG GTCACCATAA CTTGATGAGA CTATAAATGC TTAAGGTTGA<br>GAAGTTGGAT TCTTAACTCC GTTACTTAAA AAAAAACTAA GCATCTACTG TCTTCTTTCA<br>**R**<br>GCAAATGCCA AGAAATACAG ATTTCAGAAA GCTTATAAAA CTTTGGCTAT TTAGAGAAAA<br>TGACATCCTT ACTGCTTTGA GTAAGCAAAC AATTGGTTAA CAAAGTGTGA TTACTGTAAT<br>GCTTACTTGA TTCCTTAGGA GAGACTCTCA TGTGTGCTCC AGATTGGTTT GGAGGACTTT<br>GCACAATACA ACTCAGGGAG GCGTAATTTA CCAAGGCCAA AAGAGTAGGC TTGGCTTTTA<br>AGTATGCCAC AGACAATACT TTTTTCAAAT TCTACTACTA TGACTTTTTT AACTTTGTGA |
| SNP_A-2010642 | rs4620711 | A (forward) | G (forward) | HSD17B12 | TCACAACAAT TTTGTAAAGA TTTGGGAAAG AATAATAAAT ATACTACCAA ATTTATATTT<br>AATGTTTATT AAATATGGCA GAGTGTTCAG TTCCCTGTAA CTCCCTGTAA TTTCCTGATA<br>CCACAGATGA AGAAAGGTTA GCCTGTTACC AATACTATTC AAGGACATCT TTTTGATCTC<br>AGTGCACTAT ACATTTTCAA AACTAGTATG ACAGCCCTGC TATCATAACT CTGTAACCAG<br>CTTTGTCTAA CCAGCCTTGG TTTATTTTCA ATCTAGCATT TTTAGATGTA TAGCACTTGA<br>**R**<br>GTTTCAGAAG AAGGAGAAAA AACAGTTCAC AAATTATTTT GCAAAGTAGA ACAAAAAATA<br>GAAAACTAAA GCAAAACATC TGACCCCCCA ATTGAAATAC ACCAGAACCT CAAGGAAAAA<br>AGTATTCACT CATGAAATAT TATAGTGCAA ATACACTTTT GAGCAAATGA AGTGATACAT<br>GCACACATAT ACATACACAC GACTAATAAG GTAAACTGTG AAAAAGCCCA CCAGAATGAA<br>TACAAGAAAT ACTATTAGAG TTCCAAAAGT GCACAGTCAC TATCATCTAG AAAGCTCTCA |
| SNP_A-4260850 | rs10838166 | C (forward) | G (forward) | HSD17B12 | AGCATTTAAT CAGCCTGTAT TATGCAGCAT AGATGTCAAA GATACTTGAA GGGATTTCCA<br>CTATCCAGCT TGAAGGGATG CTATCTAACC AAACCTGGAT AAATTGAGCA TTAAAATTAA<br>AAGGATGGAA ACAAATTATA ATCTGTTAAT TAAAATAGAA AACCATGGCC GGGTACGGTG<br>GCTCACTCCT GTAATCCCAA CACTTTGAGA GCCCAAGGCA GGAGGATTAC CTGAGGTCAG<br>GAGTTCAAGA TGAGCCTGGT CAACATAGTG TAACCCCGTT TACTAAAACT ACAAAAGTTA<br>GCTGGGTGTG GCACGAGCCT GTAGTCCCAG CTACTTGTGA GGCTGAGGCA GGAGAATTGC<br>TTGAACCGGG AGGCAAAGGT TGCAGTGAGC CAAGATTGCG CCACTGCACT CCAGCCTGGG |

(continued)

| Marker | Synonym | Allele 1 | Allele 2 | Genomic locus | Flanking sequences |
|---|---|---|---|---|---|
| | | | | | TGGCAGAGAG AGACTCTGTC TCAAAAAAAA AAAAAAAATA GGAAAGCATG AGCCCATTGT<br>AATTATGATA AGTAAATGAA TGAAATGAAA TTTTTACAAG GAATGGGATT TAATGTAGTC<br>TTAAAGTATC TTCCTACAGA ATACAAAAAC TTGCAAATTA AAAAGGAAAT AATAGTAACT<br>TTTATAGTGG AGGAAACTGG CAGACACTAC CTTAATAAGA GACAAAAGTT ATCATCATCA<br>GTACTGGGAC AAATCAAAAT GATGTGCCAC CTGATGGGAT GCAGTGAGAA ACACAGCATC<br>ACTTCTGTGA TATTCTGGCC AAAAGTGCAT ATCCTGAATC TAATCATGAG GAAGCATACA<br>AACCTAAGTT GAGGGACATT CTACAAAGTA ATTGGCCTGT AATAATCAAT GTATCAAGGA<br>TATGAAGGTC AAGGACAGGG CAGAAAGACT GTTCCAGATT GAAGGAGGCT AACCAGAATG<br>GTAGCTAAAT GCATTGGGTG ATTCTGACTT GGATCCTTTT GCAGTAAAGG ATATTGTTGG<br>GACAACTAGT AAAAATTGAC AGGCTCTGAG GATTAGAAGA TAGTAATTAT TCATCAATAG<br>TAATTATTCC TCAATGTTAA TTTCTTGATT TCGATGGTTA CATCATTTAG GAGACTGCAG<br>TAAGTACATA CTAAGGCAGC ATTCTCAAAC TTTTTAGTCT CAGAGCCCTT TTTACTCTTA<br>AATATTATTG AGGACTCCAA AGAGCTTTTT TTTTTTTTTT ACTTTTTTTT TTAAATTATA<br>CTTTAAGTTC TGGGATACAC GTGCAGAACA TGCAAGTTTG TTACATAGGT ATACACGTGC<br>CATGGTGGTT TGTTCCACCC ATCAACTCGT AATCTACATT AGGTATTTCT CCTAATGCTA<br>TCCCTCTGCT TGCCACCCAC ACCCTGAACA GGCCCTGGTG TGTGATGCGC CCTTCCCTGT<br>GTCCATGTGT TCTCATTCTC ATTGTTCAAC TCCCACTTAT GAGTGAGAGC ATGCGGTGTT<br>TGGTTTTCTG TTCTTGTGTT AGTTTGCTGA GAATGATGGT TTCCAGCGTC ATCCATGTCC<br>CTGCAAAGGA CATGAACACA TCCCCAAAGA GCGTTTGTTT ACATAGGATA CCACTATCAA<br>TGTTTATCAC ATTATAAATT GAAACTGAGA AACTTAAAAA ATAATTATTT AACTCACTTA<br>AAAATAATAA CAGTAAACCC TTTACATGTT AACACTACAC ATTTTGAGGG AAAATAACTA<br>TATATTCTGA AACAAAACAA AATATAGTGA GAACAACCAC AT<br>**S**<br>ATTTTACATT TACAAAATCT CTTTAATATC TGGCATTGTA GCAGGATGAG CCACAGACAA<br>AACTCCTCAG ACACCGGATT AAAGAAGGAA GAGGATTTAT TCGGCCAGGA GCATCAGCAG<br>ACTTGCGTCT TAAGAGCTGA GCTCCCTCAA AAAGAAATTC TTGGCCCTTT TAAGGGCTTA<br>CAACTCCTAA GGGGTCCACA TGAAAGAGTC ATGATAGATC GAGTAAGCAT GGGAAACGTT<br>ACTGGGGGCT ACATGCATAA GCTAACAGAA CAGAAAGTTT TGCAATGCTT TTTCATACAA<br>TGTCTGGAAT TTACAGATAA CACAAGTAGT TTAGGTCAGG GATTGATATT ATTATTATTA<br>TTATTATTAT TATTATTATT ATTATTATTA TTTTAACTGC CAGGGCCAGA TGGTGACACC<br>AGGCCATCTG GCTATTTATC TTCTTTTTAA CTTTTTGCTT TTTTCTTTTC CTCCTGTCTT<br>ATAAACTAGA CAAGGGCAGG TGGGGGTGGG GGGCAGCAGG AGAAATGGTG GTCTCCTTCC<br>TTATTCCCCC CTTGGAGAAT TTCACTTATT AGTTGAAGTT CTCACTTTTA TTTTTACTTT<br>CTGAGTCTCT TTGTGAGACA GAGTGATAGT GATTCATGTA ATACACTTGT GCTGAAGTTT<br>TCTGATGAAC CAGGGTGGCA ACAAAACCTT TTATCATTTG AAGGAGCAAG GGTAATACGC<br>AGGGGAGCAG CAAGCAAGTT CTTATTACTA GCAGTATACT TACAATGAGG GTTTTAAATC<br>TTCTTATAGC TGGAAACTAT TTTTCTAAAT AAAGACTTAG GATTAAACCT GTGCTAAACC<br>TGTACAGACA CATGTGCCAA CTTTATCATG TTCTAAGCAG GTTTGTTCTT TTACTGGGTC<br>TTAAAAGCCT TTTTTTAGTG AGCAGCATAG TATTTCTTAA TAACAGAACT TTTTAAACGC |

(continued)

| Marker | Synonym | Allele 1 | Allele 2 | Genomic locus | Flanking sequences |
|---|---|---|---|---|---|
| | | | | | CAGACACTGG AACTTGTGAG CATCTGTTTG GGGAAAGAGT CAGTTAAATT AAAGTTATCT TGAGGCATTA ACTTTTTTGC TTCTTAAGGC TATTGGTCTC TCATGTTAGC TTTTCTATAA ACATAACATG AGGAAATGCT TAGGCTGCTG ACAGTGTTTT CAGCCAGCTA AGTAAACAGG GTTTTTTTTT TTGCTAAATG AGGAGGCTCT GGTAACTTCT GGTTTATATG CTTAAAGAAT GACTTAAAGA CTCAGAATTG TTGCTGGGCC AGACAGGTTC GGGTTACTTG ACCAAGTAGT GTGTGTACAG TGGGGACATC TTTCTATAGG TGTTTCTTCT AGCATGGTTA AGGGGGATAA CAACAACAAA ACAATATACA GCATATTCAT ATCTAGCAAG GACAAAAGAG GTCCTTACCT GGGAAAAAGG TTGAATACAG CGACAGAACA ATAGGAAAAC AGTTAATATT ACATGAAAAC TATTAGTCTT AAGATTTTTA ACTACATTTA CTTGCTTGAT GAGTCCTCAA GCTTTGGCTG TGCATAGACT AGTCAGCTTC CAGGATGTGA CTAGAGCAGA GCTTGCTCTA GTCCAAACAG TTTAGTCTAA AACATAGTTA GATTTGCATA TCTACTTCTG CATTCAGTCT GCTGCAATTT GTTATTTTGG TTGAAGTAGA AGGAAATCTG GCTTTACATG GATGCGTAGT TGGAGAAGAG AAAGCTATTT TAATAGCTTC TTTGGATGAT TGTGGATATT CTTTTGGTCC ATCAAAACAT TCTAAGTGAA AGCTTCTTAA AAGTTAGTTG CAATATGGAA TCTGAAATTA TATCAATTTT TTACACTGAG TGACATTAAA ATATTAAAGT TCATTGTTCT ACCTCATAGT CTGAATAGGT CTTTTATCCA TCATGACTTT GTAACTTTAT GCATTGATCA CTTGGAAAAT ATTTGTTCCT GAATTTTGCA GCTCTTCCAA ATGTTGACAC AGTATCAAAG ATAATCACAA TTGTTAATAT TACCCCCAAT CTTATCAGAA |
| SNP_A-4239787 | rs4573056 | A (forward) | C forward) | | GCATCATGCT ATCAGCCCAT AGCAATAGAC ATTATAATAC AAACTTTCAG GAAAAAAATT TATGTGTGTC TATGTATATA TTATTGAATA TATTTTTAAG TTGTCATGTC AATTATATTA CCTTAAAAAT TCAAAAATTA AGTGCATAAG ACAACGCTTA TTTTATTATA TTTACTGATC CTCTGGGTCA AAAATTTAAA TATTGCACAA TGATACTTCT CTCCTTTTCA TAATATCTTG TACTACAACT GTGAAGACCT AAATAATTGG AGTGATTCAA AAAGCTGAGA GCTAGAAAGA TCTGGAGACT TCTTTTCACA TATTTGTGTT GCCTGGTTTT ATGTGACTTA AAGAATGAGC TCAGCTGGAG TTATTGGCCA GAATGCCCCA TGTGAAAAAT TTGCCATGTG GCTTGGTGTT CCTCATAGCA TGGTTGTCTC AGGGTAGTCA TGTTTCATAT GATGGCTCAG GGCTCCAAGA GGGAGCATTC CAGTGAATAA<br>**M**<br>GTGTAGTTTT CGTGTGCTTT CCTAATATAG CTTTAAGAAT CATGCGTGAC TATCTCTGTC ACATGCTATT AGATGTAGTA ATTTACTAAG ACCAGGCCAT ACTCAATGTT AGGAGAATTA GACCTGACCT CTTACCTGAG GTAAGAGGTC AGGTCTATTT CTGTCAGAAA TAGAAATGTT TTTTATTCTG TCAAGTGATT CAAGTTTTAT TCATGAATTA TATAACTGCT AAATACCTAC TTTTGCAAAA ACTATTCAGC ATATTTCTTG TAAGTACATA GAAATTCATA GAATATATTT TGTGTACAAT ATTTTGTAAG AGAACCAACA TATT |

(continued)

| Marker | Synonym | Allele 1 | Allele 2 | Genomic locus | Flanking sequences |
|---|---|---|---|---|---|
| SNP_A-2213961 | rs10755496 | A (forward) | C forward) | | GTGTCTTAAG ACCTGAGGAC TGTAAAATGA TAGGATGAAT CCATCTAAAT GATCAGACAG<br>AATAGTTTTG TGTGACTGTT TTTTGCATGG GATTATTGTT GTCATTTTGC TTATTCATTT<br>TGGACTGAGA AATTAACTTT GTGTTTCTTT TGCTTATTGA ATCCATGGGA TTAAAGATCC<br>AGCTTGATTA AATGGACATG GAATTTTTGA TGTTTGGAAG ATAAGTATTG TTCATACCAA<br>AAATCAGAAA TTATCTATCA TTGTGTAACT AGTTGCACAT TTCTCCTAAC CCTTGTTTTA |
| | | | | | **W**<br>AAGTCATCCT CTAAACTGTT AGGAGGAAAA TAATCCCTTC TCTGGAAACT TTATCCTTTA<br>CAGTGTTTAG CACTTAAATG CAATTAAGCA AACATAATAT CCTCTTTGAA GCAAAATAGT<br>TGCTCAGCTT AAGTAACTGA AACTAATTAT CTCTGTTGAA TGAAAGACAT ATTCGGCAAG<br>TGTTTGAAGA ATGCTTCCTT CATGTTGAGC AGAAATTAGC ACTTTTATTG GCGTTGCAGG<br>TGGAGGAGCA CTCAACATGC TGTATCTGTC AGTTCATACC CACATGTAGA CCTACAAGTA |
| SNP_A-2299781 | rs7742750 | A (forward) | G (forward) | | TTGAACCAAT GACTTCCAAA CATGCCTATA ATATTTAAGT GTGCAGCTTC CTGTACCTAC<br>CTCAGGCCCA GTGTATAAGA TCCTGGAAAT CTGTATTTAT TATTCCCATA GTGACTATAT<br>TATTAGGAAA GTCTTAGAAA CACTAGGTAA ACCAGACATT CAAAGAGCTT GGTATTCAGA<br>TTATGACAAT TCAACCTTTG<br>**R**<br>TACAAAATAT TTAGGCATCA TGCTATCAGC CCATAGCAAT AGACATTATA ATACAAACTT<br>TCAGGAAAAA AATTTATGTG TGTCTATGTA TATATTATTG AATATATTTT TAAGTTGTCA<br>TGTCAATTAT ATTACCTTAA AAATTCAAAA ATTAAGTGCA TAAGACAACG CTTATTTTAT<br>TATATTTACT GATCCTCTGG GTCAAAAATT TAAATATTGC ACAATGATAC TTCTCTCCTT<br>TTCATAATAT CTTGTACTAC AACTGTGAAG ACCTAAATAA TTGGAGTGAT TCAAAAAGCT<br>GAGAGCTAGA AAGATCTGGA GACTTCTTTT CACATATTTG TGTTGCCTGG TTTTATGTGA<br>CTTAAAGAAT GAGCTCAGCT GGAGTTATTG GCCAGAATGC CCCATGTGAA AAATTTGCCA<br>TGTGGCTTGG TGTTCCTCAT AGCATGGTTG TCTCAGGGTA GTCATGTTTC ATATGATGGC<br>TCAGGGCTCC AAGAGGGAGC ATTCCAGTGA ATAAAGTGTA GTTTTCGTGT GCTTTCCTAA<br>TATAGCTTTA AGAATCATGC GTGACTATCT CTGTCACATG CTATTAGATG TAGTAATTTA<br>CTAAGACCAG GCCATACTCA ATGTTAGGAG AATTAGACCT GACCTCTTAC CTGAGGTAAG |

Table 3: Genetic markers

| Marker | Frequency of patients in the population with Allele 1 | Frequency of patients in the population with Allele 2 | Genotype in patient population | Genotype in patient population | Genotype in patient population |
|---|---|---|---|---|---|
| SNP_A-2040233 | C (62%) | T (85%) | CC (15%) | CT (47%) | TT (38%) |
| SNP_A-1978609 | A (55%) | T (87%) | AA (13%) | AT (42%) | TT (45%) |
| SNP_A-1986846 | C (48%) | G (93%) | CC (7%) | CG (41%) | GG (52%) |
| SNP_A-2223123 | C (59%) | A (87%) | AA (41(%) | AC (46%) | CC (13%) |
| SNP_A-2085239 | T (34%) | G (96%) | TT (4%) | GT (30%) | GG (66%) |

REFERENCES

1. Anderson, M. E. and Siahaan, T. J. (2003). Targeting ICAM-1/LFA-1 interaction for controlling autoimmune diseases: designing peptide and small molecule inhibitors. Peptides 24:487-501.

2. Binz, H. K., Amstutz, P., Kohl, A., Stumpp, M. T., Briand, C., Forrer, P., Grutter, M. G., and Pluckthun, A. (2004). High-affinity binders selected from designed ankyrin repeat protein libraries. Nat Biotechnol 22 :575-582.

3. Chen, X., Zehnbauer, B., Gnirke, A., and Kwok, P. Y. (1997). Fluorescence energy transfer detection as a homogeneous DNA diagnostic method. Proc Natl Acad Sci U S A 94:10756-10761.

4. Di, X., Matsuzaki, H., Webster, T. A., Hubbell, E., Liu, G., Dong, S., Bartell, D., Huang, J., Chiles, R., Yang, G., Shen, M. M. , Kulp, D., Kennedy, G. C., Mei, R., Jones, K. W., and Cawley, S. (2005). Dynamic model based algorithms for screening and genotyping over 100 K SNPs on oligonucleotide microarrays. Bioinformatics 21 : 1958-1963.

5. Docherty, S. J., Butcher, L. M., Schalkwyk, L. C., and Plomin, R. (2007). Applicability of DNA pools on 500 K SNP microarrays for cost-effective initial screens in genomewide association studies. BMC Genomics 8:214.

6. Gill, D. S. and Damle, N. K. (2006). Biopharmaceutical drug discovery using novel protein scaffolds. Curr Opin Biotechnol 17:653-658.

7. Gingeras, T. R., Richman, D. D., Kwoh, D. Y., and Guatelli, J. C. (1990). Methodologies for in vitro nucleic acid amplification and their applications. Vet Microbiol 24:235-251.

8. Kennedy, G. C., Matsuzaki, H., Dong, S., Liu, W. M., Huang, J., Liu, G., Su, X., Cao, M., Chen, W., Zhang, J., Liu, W., Yang, G., Di, X., Ryder, T., He, Z., Surti, U., Phillips, M. S., Boyce-Jacino, M. T., Fodor, S. P., and Jones, K. W. (2003). Large-scale genotyping of complex DNA. Nat Biotechnol 21:1233-1237.

9. Kim, S. and Misra, A. (2007). SNP genotyping: technologies and biomedical applications. Annu Rev Biomed Eng 9:289-320.

10. Lin, S., Carvalho, B., Cutler, D. J., Arking, D. E., Chakravarti, A., and Irizarry, R. A. (2008). Validation and extension of an empirical Bayes method for SNP calling on Affymetrix microarrays. Genome Biol 9:R63.

11. Matsuzaki, H., Dong, S., Loi, H., Di, X., Liu, G., Hubbell, E., Law, J., Berntsen, T., Chadha, M., Hui, H., Yang, G., Kennedy, G. C., Webster, T. A., Cawley, S., Walsh, P. S., Jones, K. W., Fodor, S. P., and Mei, R. (2004). Genotyping over 100,000 SNPs on a pair of oligonucleotide arrays. Nat Methods 1:109-111.

12. Mosavi, L. K., Cammett, T. J., Desrosiers, D. C., and Peng, Z. Y. (2004). The ankyrin repeat as molecular architecture for protein recognition. Protein Sci 13:1435-1448.

13. Nickerson, D. A., Ankener, W., Delahunty, C., and Kwok, P. Y. (2001). Genotyping by ligation assays. Curr Protoc Hum Genet Chapter 2:Unit.

14. Nilsson, F. Y. and Tolmachev, V. (2007). Affibody molecules: new protein domains for molecular imaging and targeted tumor therapy. Curr Opin Drug Discov Devel 10:167-175.

15. Rabbee, N. and Speed, T. P. (2006). A genotype calling algorithm for affymetrix SNP arrays. Bioinformatics 22: 7-12.

16. Ruano, G., Deinard, A. S., Tishkoff, S., and Kidd, K. K. (1994). Detection of DNA sequence variation via deliberate heteroduplex formation from genomic DNAs amplified en masse in "population tubes". PCR Methods Appl 3:225-231.

17. Ruano, G. and Kidd, K. K. (1989). Direct haplotyping of chromosomal segments from multiple heterozygotes via allele-specific PCR amplification. Nucleic Acids Res 17:8392.

18. Ruano, G. and Kidd, K. K. (1991). Genotyping and haplotyping of polymorphisms directly from genomic DNA via coupled amplification and sequencing (CAS). Nucleic Acids Res 19:6877-6882.

19. Sillerud, L. O. and Larson, R. S. (2005). Design and structure of peptide and peptidomimetic antagonists of protein-protein interaction. Curr Protein Pept Sci 6:151-169.

20. Tian, F., Wu, Y., Zhou, Y., Liu, X., Visvikis-Siest, S., and Xia, Y. (2008). A new single nucleotide polymorphism genotyping method based on gap ligase chain reaction and a microsphere detection assay. Clin Chem Lab Med.

21. Tomlinson, I. M. (2004). Next-generation protein drugs. Nat Biotechnol 22:521-522.

22. Wang, L., Luhm, R., and Lei, M. (2007). SNP and mutation analysis. Adv Exp Med Biol 593:105-116.

23. Xingyuan, M., Wenyun, Z., and Tianwen, W. (2006). Leukocyte function-associated antigen-1: structure, function and application prospects. Protein Pept Lett 13:397-400.

**Claims**

1. A method of genotyping an individual afflicted with psoriasis comprising detecting the presence or absence of an allele of at least one of the genetic markers selected from the group consisting of SNP_A-2040233 (rs4043263), SNP_A-1978609 (rs17501861), SNP_A-1986846 (rs9294506), SNP_A-2223123 (rs6485472), SNP_A-2085239 (rs11251116), SNP_A-4269573 (rs11037653), SNP_A-2010642 (rs4620711), SNP_A-4260850 (rs10838166), SNP_A-4239787 (rs4573056), SNP_A-2213961 (rs10755496) or _A-2299781 (rs7742750) in a biological sample of the individual.

2. The method according to claim 1 wherein the allele is detected on both of paired chromosomes.

3. The method according to claim 1 or 2, wherein the presence of absence of the alleles of at least two genetic markers are detected.

4. The method according to any one of claims 1, 2 or 3 wherein the allele(s) detected is (are):

   a) thymine ("T") of the marker SNP_A-2040233 (rs4043263);
   b) adenine ("A") of the marker SNP_A-1978609 (rs17501861);
   c) cytosine ("C") of the marker SNP_A-1986846 (rs9294506);
   d) adenine ("A") of the marker SNP_A-2223123 (rs6485472);
   e) thymine ("T") of the marker SNP_A-2085239 (rs11251116);
   f) guanine ("G") for the marker SNP_A-4269573 (rs11037653);
   g) guanine ("G") for the marker SNP_A-2010642 (rs4620711);
   h) guanine ("G") for the marker SNP_A-4260850 (rs10838166);
   i) cytosine ("C") for the marker SNP_A-4239787 (rs4573056);
   j) adenine ("A") for the marker SNP_A-2213961 (rs10755496), or
   k) cytosine ("C") for the marker SNP_A-2299781 (rs7742750).

5. A kit comprising means for performing the method according to any of the claims 1 to 4 and instructions for use.

6. A kit according to claim 5 comprising a specific primer or probe for detecting the presence or absence an allele.

7. A method of diagnosis to identify an individual afflicted with psoriasis that is more likely to exhibit a very good response, a response or a partial response to the treatment of psoriasis with an inhibitor of LFA-1 as assessed by the PGA score comprising genotyping an individual afflicted with psoriasis according to any of methods of claims 1 to 4.

8. A method of treating psoriasis

   a) genotyping an individual afflicted with psoriasis according to any one of the methods of claims 1 to 4,
   b) selecting an individual afflicted with psoriasis based on the allele of said marker that is detected in the individual, and

c) administering to said selected individual a therapeutically effective amount of an inhibitor of LFA-1.

**9.** The method of treating psoriasis according to claim 8, wherein the individual has not previously been treated with an inhibitor of LFA-1.

**10.** The method according to any one of claims 7 to 9, wherein the inhibitor of LFA-1 is an inhibitor of CD11a.

**11.** The method according to claim 9 or 10, wherein the inhibitor is a peptide or protein specifically binding to LFA-1 or CD11 a, preferably an antibody or antigen binding fragment thereof binding specifically to LFA-1 or CD11a, more preferably efalizumab.

**12.** An inhibitor of LFA-1 for use in the treatment of psoriasis **characterized in that** the treatment comprises

a) genotyping an individual afflicted with psoriasis according to any one of the methods of claims 1 to 4,
b) selecting an individual afflicted with psoriasis based on the allele of said marker that is detected in the individual, and
c) administering to said selected individual a therapeutically effective amount of an inhibitor of LFA-1.

**13.** An inhibitor of LFA-1 for use in the treatment of an individual afflicted with psoriasis **characterized in that** the individual has one allele or any combination of the allele(s) selected from the group consisting of thymine ("T") for the marker SNP_A-2040233 (rs4043263), adenine ("A") for the marker SNP_A-1978609 (rs17501861), cytosine ("C") for the marker SNP_A-1986846 (rs9294506), adenine ("A") for the marker SNP_A-2223123 (rs6485472), thymine ("T") for the marker SNP_A-2085239 (rs11251116), guanine ("G") for the marker SNP_A-4269573 (rs11037653), guanine ("G") for the marker SNP_A-2010642 (rs4620711), guanine ("G") for the marker SNP_A-4260850 (rs10838166), cytosine ("C") for the marker SNP_A-4239787 (rs4573056), adenine ("A") for the marker SNP_A-2213961 (rs10755496) or cytosine ("C") for the marker SNP_A-2299781 (rs7742750).

**14.** An inhibitor of LFA-1 for use in the treatment of an individual afflicted with psoriasis according to claim 13, wherein the individual has not previously been treated with an inhibitor of LFA-1.

**15.** An inhibitor of LFA-1 for use in the treatment of an individual afflicted with psoriasis according to claim 13 or 14, wherein at least one of the allele(s) is present on both paired chromosomes of said individual.

**16.** An inhibitor of LFA-1 according to any one of claims 12 to 15, which is an inhibitor of CD11a.

**17.** An inhibitor of LFA-1 according to any one of claims 12 to 15 or an inhibitor of CD11 according to claim 16, which is a peptide or protein specifically binding to LFA-1 or CD11 a, preferably an antibody or antigen binding fragment thereof binding specifically to LFA-1 or CD11a, more preferably efalizumab.

**Figure 1:**

**PSORIASIS AREA AND SEVERITY INDEX - PASI**

PASI Date: Day Month Year

**SEVERITY OF PSORIATIC LESIONS**

Circle one number in each of the categories below:

0 = None 1 = Slight 2 = Moderate 3 = Severe 4 = Very Severe

| | | Head | Trunk | Upper Limbs | Lower Limbs |
|---|---|---|---|---|---|
| 1 | Erythema | 0 1 2 3 4 | 0 1 2 3 4 | 0 1 2 3 4 | 0 1 2 3 4 |
| 2 | Thickness | 0 1 2 3 4 | 0 1 2 3 4 | 0 1 2 3 4 | 0 1 2 3 4 |
| 3 | Scaling | 0 1 2 3 4 | 0 1 2 3 4 | 0 1 2 3 4 | 0 1 2 3 4 |
| 4 | **Total each column** | | | | |

**AREA OF PSORIATIC INVOLVEMENT**

| | | | | | |
|---|---|---|---|---|---|
| 5 | Degree of involvement | 0 = None<br>1 = < 10% | 2 = 10 to < 30%<br>3 = 30 to < 50% | 4 = 50 to < 70%<br>5 = 70 to < 90% | 6 = 90 to 100% |
| 6 | Insert degree of involvement from Row 5 | | | | |
| 7 | Multiply Row 4 by Row 6 | | | | |
| 8 | | x 0.10 | x 0.30 | x 0.20 | x 0.40 |
| 9 | Multiply Row 7 by Row 8 | . | . | . | . |

**TOTAL PASI SCORE** . **(Add together each column in Row 9) Minimum 10.0 for study inclusion.**

**Assessor:** ________________________________ (print name)

Figure 2:

(a)

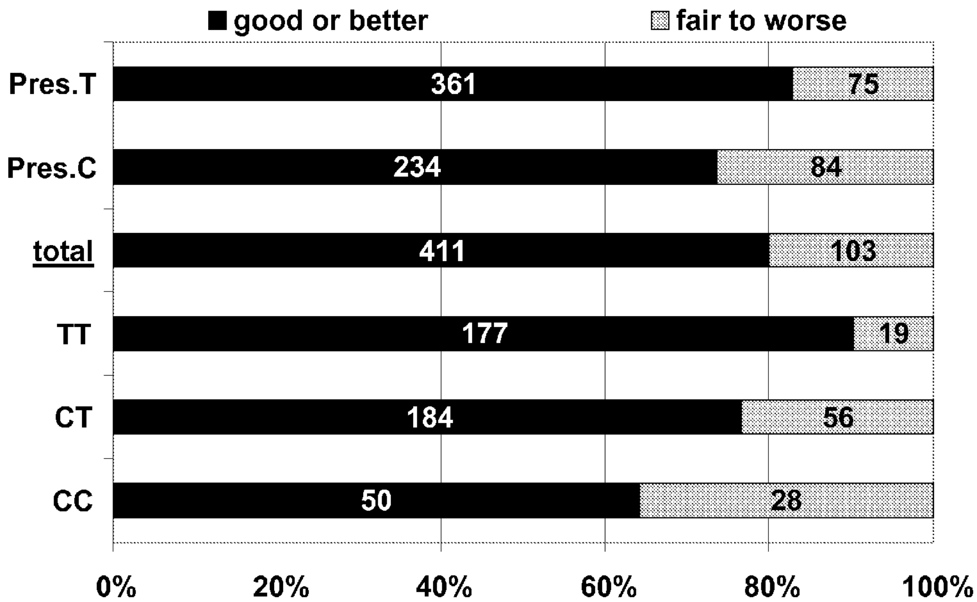


(b)

| SNP | pvalue | OR | OR_low | OR_up | RR_min | RR_min_low | RR_min_up | RR_maj | RR_maj_low | RR_maj_up |
|---|---|---|---|---|---|---|---|---|---|---|
| SNP_A-2040233 | 2.59E-07 | 2.26 | 1.66 | 3.08 | 1.57 | 1.35 | 1.84 | 0.70 | 0.60 | 0.82 |

Figure 3:

(a)

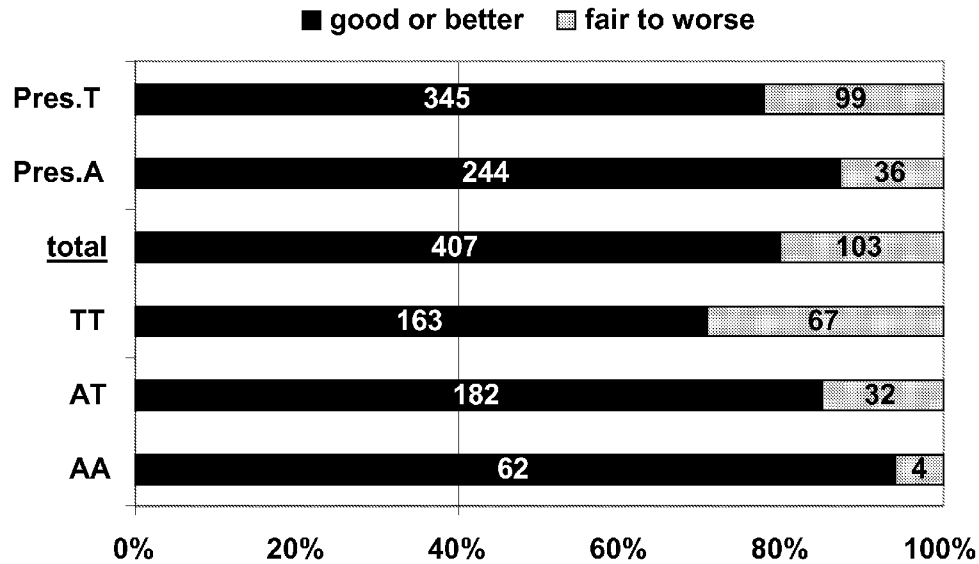


(b)

| SNP | Fisher | OR | OR_low | OR_up | RR_min | RR_min_low | RR_min_up | RR_maj | RR_maj_low | RR_maj_up |
|---|---|---|---|---|---|---|---|---|---|---|
| SNP_A-1978609 | 4.31E-07 | 0.40 | 0.28 | 0.58 | 0.52 | 0.39 | 0.69 | 1.29 | 1.19 | 1.41 |

Figure 4:

(a)

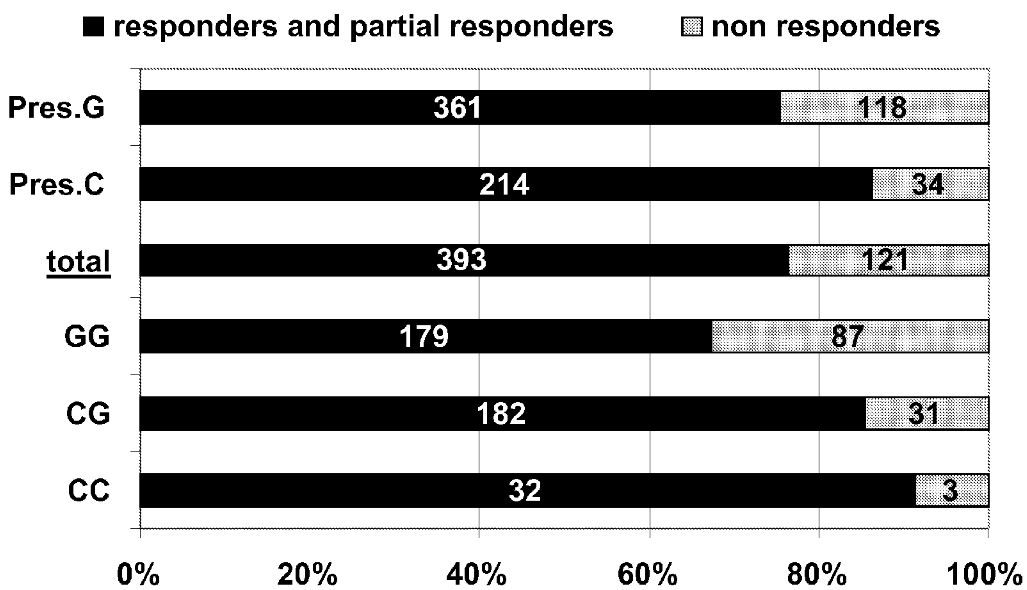


(b)

| SNP | pvalue | OR | OR_low | OR_up | RR_min | RR_min_low | RR_min_up | RR_maj | RR_maj_low | RR_maj_up |
|---|---|---|---|---|---|---|---|---|---|---|
| SNP_A-1986846 | 4.53E-07 | 2.52 | 1.72 | 3.70 | 2.05 | 1.50 | 2.80 | 0.81 | 0.76 | 0.87 |

Figure 5:

(a)

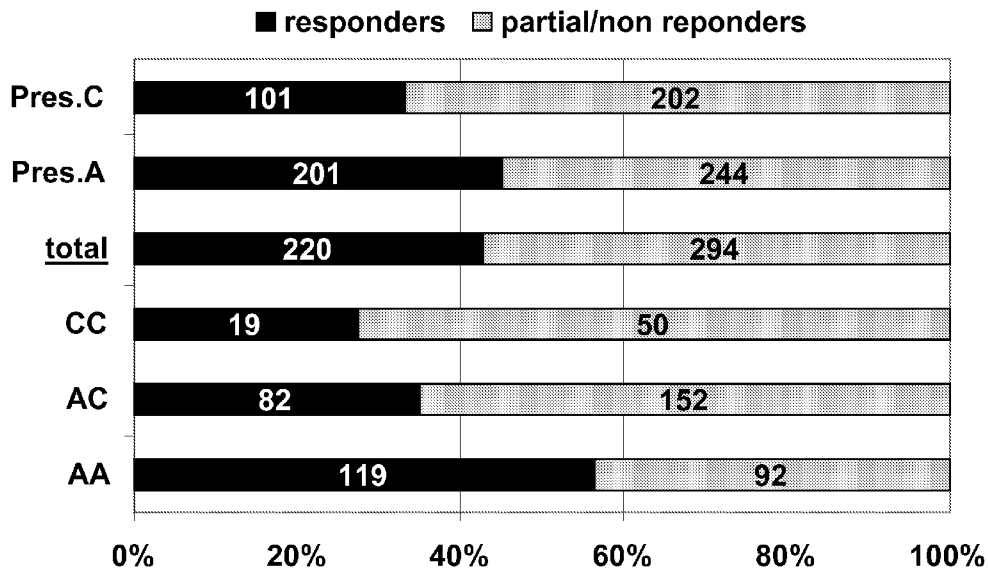


(b)

| SNP | pvalue | OR | OR_low | OR_up | RR_min | RR_min_low | RR_min_up | RR_maj | RR_maj_low | RR_maj_up |
|---|---|---|---|---|---|---|---|---|---|---|
| SNP_A-2223123 | 2.89E-07 | 0.50 | 0.38 | 0.65 | 0.64 | 0.53 | 0.76 | 1.27 | 1.16 | 1.39 |

Figure 6:

(a)

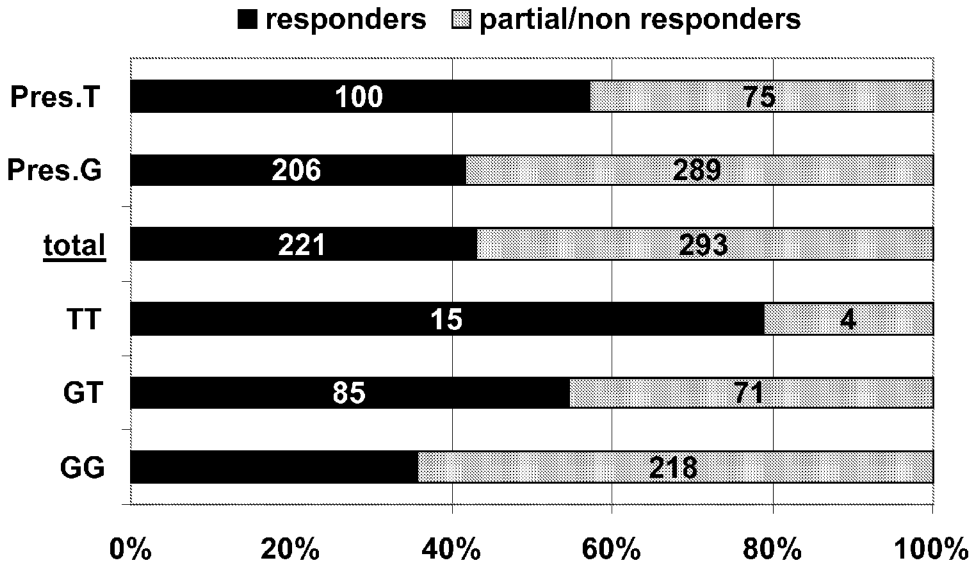


(b)

| SNP | Fisher | OR | OR_low | OR_up | RR_min | RR_min_low | RR_min_up | RR_maj | RR_maj_low | RR_maj_up |
|---|---|---|---|---|---|---|---|---|---|---|
| SNP_A-2085239 | 5.06E-07 | 2.26 | 1.64 | 3.10 | 1.93 | 1.49 | 2.50 | 0.86 | 0.80 | 0.91 |

Europäisches Patentamt
European Patent Office
Office européen des brevets

# EUROPEAN SEARCH REPORT

Application Number
EP 08 16 1361

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| E | WO 2008/124937 A (NEWLAB [CA]; GULLIVER WAYNE [CA]) 23 October 2008 (2008-10-23)<br>* claims 1-9,15-28,46,47 *<br>* page 2, paragraph 3 - page 6 *<br>* page 14, line 13 - page 19, line 8 *<br>----- | 1-17 | INV.<br>C12Q1/68 |
| X | COSTANZO ET AL: "Abstracts for the International Investigative Dermatology 2008"<br>JOURNAL OF DERMATOLOGICAL SCIENCE, ELSEVIER SCIENCE PUBLISHERS, SHANNON, IR,<br>vol. 50, no. 2, 1 April 2008 (2008-04-01), pages e1-e285, XP022575973<br>ISSN: 0923-1811<br>* paragraph [0416] *<br>----- | 1-17 | |
| X | TEJASVI T ET AL: "Initial pharmacogenomic assessment of TNF-alpha and TRAF1 polymorphisms in psoriasis: response to TNF blockers and other systemic therapies"<br>JOURNAL OF INVESTIGATIVE DERMATOLOGY,<br>vol. 128, no. Suppl. 1,<br>April 2008 (2008-04), page S74, XP008098944<br>INTERNATIONAL INVESTIGATIVE DERMATOLOGY MEETING; KYOTO, JAPAN; MAY 14 17, 2008<br>ISSN: 0022-202X<br>* paragraph [0442] *<br>-----<br>-/-- | 1-17 | TECHNICAL FIELDS SEARCHED (IPC)<br>C12Q |

~~The present search report has been drawn up for all claims~~

1

| Place of search | Date of completion of the search | Examiner |
|---|---|---|
| The Hague | 15 December 2008 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 08 16 1361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GULLIVER W ET AL: "HLA-Cw6 and TNF-alpha polymorphisms may help predict response to biologic therapy in patients with chronic plaque psoriasis" JOURNAL OF INVESTIGATIVE DERMATOLOGY, vol. 127, no. Suppl. 2, October 2007 (2007-10), page S22, XP008098945 37TH ANNUAL MEETING OF THE EUROPEAN-SOCIETY-FOR-DERMATOLOGICAL-RESEARCH; ZURICH, SWITZERLAND; SEPTEMBER 05 -08, 2007 ISSN: 0022-202X * paragraph [0129] * ----- | 1-17 | |
| X | WO 2005/112568 A (GENAISSANCE PHARMACEUTICALS [US]; KOSHY BEENA [US]; DAIN BRADLEY J [US]) 1 December 2005 (2005-12-01) * claims 1-16 * * page 1, paragraph 23 - page 7 * * page 33, line 25 - page 35, line 4 * ----- | 5,6 | TECHNICAL FIELDS SEARCHED (IPC) |
| X | DATABASE DBSNP ncbi; 29 February 2008 (2008-02-29), "http://www.ncbi.nlm.nih.gov/SNP/snp_ref.cgi?rs=4043263" XP002505587 retrieved from NCBI Database accession no. rz4043263 * abstract * ----- -/-- | 5,6 | |

~~The present search report has been drawn up for all claims~~

| Place of search | Date of completion of the search | Examiner |
|---|---|---|
| The Hague | 15 December 2008 | Bruma, Anja |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 08 16 1361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE VLAM K ET AL: "Biological biomarkers in psoriatic disease. A review" JOURNAL OF RHEUMATOLOGY 200807 CA, vol. 35, no. 7, 1 July 2008 (2008-07-01), pages 1443-1448, XP002505586 ISSN: 0315-162X * the whole document * ----- | | |
| A | BUSSEL ET AL: "Update on Therapeutic Monoclonal Antibodies" CURRENT PROBLEMS IN PEDIATRIC AND ADOLESCENT HEALTH CARE, MOSBY, vol. 37, no. 4, 13 April 2007 (2007-04-13), pages 118-135, XP022020661 ISSN: 1538-5442 * the whole document * ----- | | |
| | | | TECHNICAL FIELDS SEARCHED (IPC) |

~~The present search report has been drawn up for all claims~~

1

| Place of search | Date of completion of the search | Examiner |
|---|---|---|
| The Hague | 15 December 2008 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 08 16 1361

| CLAIMS INCURRING FEES |
| --- |
| The present European patent application comprised at the time of filing claims for which payment was due.<br>☐ Only part of the claims have been paid within the prescribed time limit. The present European search report has been drawn up for those claims for which no payment was due and for those claims for which claims fees have been paid, namely claim(s):<br>☐ No claims fees have been paid within the prescribed time limit. The present European search report has been drawn up for those claims for which no payment was due. |
| **LACK OF UNITY OF INVENTION** |
| The Search Division considers that the present European patent application does not comply with the requirements of unity of invention and relates to several inventions or groups of inventions, namely:<br>see sheet B<br>☐ All further search fees have been paid within the fixed time limit. The present European search report has been drawn up for all claims.<br>☐ As all searchable claims could be searched without effort justifying an additional fee, the Search Division did not invite payment of any additional fee.<br>☐ Only part of the further search fees have been paid within the fixed time limit. The present European search report has been drawn up for those parts of the European patent application which relate to the inventions in respect of which search fees have been paid, namely claims:<br>☒ None of the further search fees have been paid within the fixed time limit. The present European search report has been drawn up for those parts of the European patent application which relate to the invention first mentioned in the claims, namely claims:<br>1-17 (all partially)<br>☐ The present supplementary European search report has been drawn up for those parts of the European patent application which relate to the invention first mentioned in the claims (Rule 164 (1) EPC). |

Europäisches Patentamt
European Patent Office
Office européen des brevets

## LACK OF UNITY OF INVENTION SHEET B

Application Number

EP 08 16 1361

The Search Division considers that the present European patent application does not comply with the requirements of unity of invention and relates to several inventions or groups of inventions, namely:

Invention 1: claims 1-17 (all partially)

Methods using the SNP represented by SEQ ID No.1, kits comprising said sequence, inhibitors used in the treatment of an individual characterized in that it has said SNP.

---

Invention 2: claims 1-17 (all partially)

Methods using the SNP represented by SEQ ID No.2, kits comprising said sequence, inhibitors used in the treatment of an individual characterized in that it has said SNP.

---

Invention 3-11: claims 1-17 (all partially)

Methods using the SNP represented by SEQ ID No.n, wherein n=3-11 respectively, kits comprising said sequence, inhibitors used in the treatment of an individual characterized in that it has said SNP.

---

## ANNEX TO THE EUROPEAN SEARCH REPORT ON EUROPEAN PATENT APPLICATION NO. EP 08 16 1361

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2008

| Patent document cited in search report | Publication date | Patent family member(s) | Publication date |
|---|---|---|---|
| WO 2008124937 A | 23-10-2008 | NONE | |
| WO 2005112568 A | 01-12-2005 | BR PI0510691 A | 26-12-2007 |
| | | CA 2565804 A1 | 01-12-2005 |
| | | CN 1997756 A | 11-07-2007 |
| | | EP 1747291 A2 | 31-01-2007 |
| | | KR 20070011558 A | 24-01-2007 |
| | | US 2008020383 A1 | 24-01-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9215712 A
- US 5679524 A
- WO 9102087 A
- WO 9009455 A
- WO 9517676 A
- US 5302509 A
- US 5945283 A
- US 5605798 A
- WO 8910414 A


### Non-patent literature cited in the description


- **Anderson, M. E ; Siahaan, T. J.** Targeting ICAM-1/LFA-1 interaction for controlling autoimmune diseases: designing peptide and small molecule inhibitors. *Peptides,* 2003, vol. 24, 487-501
- **Binz, H. K. ; Amstutz, P. ; Kohl, A. ; Stumpp, M. T. ; Briand, C. ; Forrer, P. ; Grutter, M. G. ; Pluckthun, A.** High-affinity binders selected from designed ankyrin repeat protein libraries. *Nat Biotechnol,* 2004, vol. 22, 575-582
- **Chen, X. ; Zehnbauer, B. ; Gnirke, A. ; Kwok, P. Y.** Fluorescence energy transfer detection as a homogeneous DNA diagnostic method. *Proc Natl Acad Sci U S A,* 1997, vol. 94, 10756-10761
- **Di, X. ; Matsuzaki, H. ; Webster, T. A. ; Hubbell, E. ; Liu, G. ; Dong, S. ; Bartell, D. ; Huang, J. ; Chiles, R. ; Yang, G.** Dynamic model based algorithms for screening and genotyping over 100 K SNPs on oligonucleotide microarrays. *Bioinformatics,* 2005, vol. 21, 1958-1963
- **Docherty, S. J. ; Butcher, L. M. ; Schalkwyk, L. C. ; Plomin, R.** Applicability of DNA pools on 500 K SNP microarrays for cost-effective initial screens in genomewide association studies. *BMC Genomics,* 2007, vol. 8, 214
- **Gill, D. S. ; Damle, N. K.** Biopharmaceutical drug discovery using novel protein scaffolds. *Curr Opin Biotechnol,* 2006, vol. 17, 653-658
- **Gingeras, T. R. ; Richman, D. D. ; Kwoh, D. Y. ; Guatelli, J. C.** Methodologies for in vitro nucleic acid amplification and their applications. *Vet Microbiol,* 1990, vol. 24, 235-251
- **Kennedy, G. C. ; Matsuzaki, H. ; Dong, S. ; Liu, W. M. ; Huang, J. ; Liu, G. ; Su, X. ; Cao, M. ; Chen, W. ; Zhang, J., Liu, W.** Large-scale genotyping of complex DNA. *Nat Biotechnol,* 2003, vol. 21, 1233-1237
- **Kim, S. ; Misra, A.** SNP genotyping: technologies and biomedical applications. *Annu Rev Biomed Eng,* 2007, vol. 9, 289-320
- **Lin, S. ; Carvalho, B. ; Cutler, D. J. ; Arking, D. E. ; Chakravarti, A. ; Irizarry, R. A.** Validation and extension of an empirical Bayes method for SNP calling on Affymetrix microarrays. *Genome Biol,* 2008, vol. 9, R63
- **Matsuzaki, H. ; Dong, S. ; Loi, H. ; Di, X. ; Liu, G. ; Hubbell, E. ; Law, J. ; Berntsen, T. ; Chadha, M. ; Hui, H.** Genotyping over 100,000 SNPs on a pair of oligonucleotide arrays. *Nat Methods,* 2004, vol. 1, 109-111
- **Mosavi, L. K. ; Cammett, T. J. ; Desrosiers, D. C. ; Peng, Z. Y.** The ankyrin repeat as molecular architecture for protein recognition. *Protein Sci,* 2004, vol. 13, 1435-1448
- Genotyping by ligation assays. **Nickerson, D. A. ; Ankener, W. ; Delahunty, C. ; Kwok, P. Y.** Curr Protoc Hum Genet. 2001
- **Nilsson, F. Y. ; Tolmachev, V.** Affibody molecules: new protein domains for molecular imaging and targeted tumor therapy. *Curr Opin Drug Discov Devel,* 2007, vol. 10, 167-175
- **Rabbee, N. ; Speed, T. P.** A genotype calling algorithm for affymetrix SNP arrays. *Bioinformatics,* 2006, vol. 22, 7-12
- **Ruano, G. ; Deinard, A. S. ; Tishkoff, S. ; Kidd, K. K.** Detection of DNA sequence variation via deliberate heteroduplex formation from genomic DNAs amplified en masse in ''population tubes''. *PCR Methods Appl,* 1994, vol. 3, 225-231
- **Ruano, G. ; Kidd, K. K.** Direct haplotyping of chromosomal segments from multiple heterozygotes via allele-specific PCR amplification. *Nucleic Acids Res,* 1989, vol. 17, 8392
- **Ruano, G. ; Kidd, K. K.** Genotyping and haplotyping of polymorphisms directly from genomic DNA via coupled amplification and sequencing (CAS). *Nucleic Acids Res,* 1991, vol. 19, 6877-6882

- **Sillerud, L. O. ; Larson, R. S.** Design and structure of peptide and peptidomimetic antagonists of protein-protein interaction. *Curr Protein Pept Sci,* 2005, vol. 6, 151-169
- **Tian, F. ; Wu, Y. ; Zhou, Y. ; Liu, X. ; Visvikis-Siest, S. ; Xia, Y.** A new single nucleotide polymorphism genotyping method based on gap ligase chain reaction and a microsphere detection assay. *Clin Chem Lab Med.,* 2008
- **Tomlinson, I. M.** Next-generation protein drugs. *Nat Biotechnol,* 2004, vol. 22, 521-522
- **Wang, L. ; Luhm, R. ; Lei, M.** SNP and mutation analysis. *Adv Exp Med Biol,* 2007, vol. 593, 105-116
- **Xingyuan, M. ; Wenyun, Z. ; Tianwen, W.** Leukocyte function-associated antigen-1: structure, function and application prospects. *Protein Pept Lett,* 2006, vol. 13, 397-400

SEQUENCE LISTING

<110> Laboratoires Serono S.A.

<120> GENETIC MARKERS OF RESPONSE TO EFALIZUMAB

<130> 1196 EP EPA

<160> 11

<170> PatentIn version 3.3

<210> 1
<211> 890
<212> DNA
<213> Homo sapiens

<220>
<221> variation
<222> (496)..(496)
<223> SNP rs4043263

<400> 1
```
tacccacgca ggggacactt agggctccag ggtttggtcc gaggctgaga ggcatctggt      60

gactctgcac tgaccatttt tttttttct tgagacagag ccttgctctg tcgccaaggc     120

tggagtgcag tggtgtgacc tcagctcact gcaacctctg cctcccgggt tcaagcaatt     180

ctctctctca gcctcccaag tagttgggac tgcaggcaag cgccaccaca cctggctaat     240

ttttgtatct ttagtagaga cagggtttca ccatgttggc caggctggtc tcgaactcct     300

gacttcaagt gatccacctg ccttggcctc ccaagtgttg ggattacagg cgtgagccac     360

cacgctcggc ctacactgac ctttgagagt cacaaaggct gagcctcacc tgtgccctct     420

ctgccttcct gccattcatc tgccagaagc caccctttcc ctatctggag ggtcagctga     480

accacacatc agaccyggat agactgggaa ctgggaactg agaaagggtt aagcatgggc     540

atggaggagt tgagaggcag aagcaaaaag aaagttaaaa ataatgttat tcagcctaga     600

gcaaggaaga caagggcaca gctagacttc agtttgtgac tgatcggttt gagaatgatt     660

cttgcttttt tccccctgca ctctgaatag gaagaagctg accttctgct gcaggagaga     720

ctggagttag atgttaccca gtgtcctgcg gcgaccagat gccagagtag gaatctaagc     780

tgaacgtggc cctgggtcct actctttcgg catgtgatcc gaggtggcct tgggaaccct     840

acctgttcag gaatgggggg ctatgatccc aaagcttcta gctgccaatt               890
```

<210> 2
<211> 601
<212> DNA
<213> Homo sapiens

<220>
<221> variation
<222> (301)..(301)
<223> SNP rs17501861

<400> 2
```
ttatacatat atatacagta tacagtatat atacatatac tgtatatatg tatattatat      60
```

gtacattgta tatatataat gtatacagta tactatatat acacacacac acacacacac 120

acacataaat acaagactat ggagacctaa catgttccct gtgtttgccc agcatttgct 180

ttcgctttta ttaaaatctg atagtttacc ctttgcctgg atttccatga gttatgagta 240

tgtccttcca atagtactta ccacaaatat aacttgattg aaagtagttt aagttgctct 300

wcaaaggtta tttctaaaat actgtgtttc tcattgagat ttctctttga gatattacct 360

aacttgatct atatattagg tagatataca atatattagg tagatataca atatctatat 420

attaggtaga tatacaatat attccaatat gacatgcaca gtacataaga taaagccctc 480

acactttcac accactaaat atatttaaac aacgtcaaat tttgaaagaa atttcaaaat 540

tcaggaccaa atagtctgat aaagacttac aataaatata ttgattttta ttataatgat 600

t 601

<210> 3
<211> 601
<212> DNA
<213> Homo sapiens

<220>
<221> variation
<222> (301)..(301)
<223> SNP rs9294506

<400> 3
tataacatct aggagtattg tttcataaaa atagtatgta aatatataac tgaattacta 60

cataatgagg aaattcccat gggcaaaaaa ttaccaggat tatgtattct gatacatgag 120

cacaggaatc tgcctgcttg ggccatctgc ctacaccatg tggtccacag cactgccttt 180

aaagggctat atgtgtaggg gacataagac aatgtctgaa gcctgcacaa agccagaacc 240

tctgaaagga gacacatgga atgaaacagg gatctagaca aacagcccta cctcgcagaa 300

sgaatttttt aaactattag taatagcaat aacttttttt tgaaaaaaaa aatacgcatc 360

tacagtcgat tgctctggac attgattctg ggtgaaaggg aagatctccc ctgataattc 420

agcacgtgat tggtcctcat ttgggtttga ggctaatatt cacattctct ttatggcttg 480

aatgatacca agtcaatctt taagaggttg aacatgttgg gagtgctttc tgagaaaatc 540

ttaggcaaat atcctcttga agaaaatatc tcaaataatt accaaaatat aaacaagaaa 600

a 601

<210> 4
<211> 601
<212> DNA
<213> Homo sapiens

<220>
<221> variation
<222> (301)..(301)
<223> SNP rs6485472

<400> 4
caggtctctc ttcccacctg cactgtggga cagtgatgag gtttctaagg aaggtagaaa 60

acagtgactt acttcaaagg agacctatta cgtcctctat gacagtagaa tttttataga 120
ggtgtggaca aggataccac aacagtctga tagaatatta aagcacttat aaaataaagg 180
cttggcaatt ttttttaat ccaggtgaca attaagggag tcttattggg aatttcacag 240
gtctgaggga attccatgag agagcatatc tataggaagc tgtaagtgtg agggactgag 300
ktttatagag ttgggcgaat ttaatgtctg gcttgtggga ggactagtaa ttaaagagaa 360
tatgtttata tgaaaaggta ctttcctgtc agcaattggt gttttctgct ttgggttttt 420
tcgcaacatt ttagttctag tagatcctca gtaatttgta ccactgagac agcattgtgt 480
ggctgttagc aagtaagcag tataatgaat taagaatgtt gcattacaga aaacattgct 540
tgacttgttt atttggatat ttgtcataac aaactagaaa atgcgctgaa gtatgagcaa 600
c 601

<210> 5
<211> 1857
<212> DNA
<213> Homo sapiens

<220>
<221> variation
<222> (1025)..(1025)
<223> SNP rs11251116

<400> 5
tgcctagagg tctcctgtcc agtaaagact agatgaagaa agtaagaact gaaaagggat 60
aaaataatga actttatgtt ttctacttgt ttttctgttt gtttaggtat ggtgtcttgc 120
tctgctgccc aagctggagt tcagtggtgc aatcatagct atgtatatta ccttttcttt 180
aaaaaagttt aacttttaga acagttttca attcccagca aaattgagaa aaaggtacag 240
gaagttctca tatattcccc agctcccaca catggacatg ctcccgccat cagcacctcc 300
cctggagggt gcatttgtta caatggatga acctacatgg acatatcact gttacataga 360
gtccacagtg caactgaggg ctcgctctgg gtattgtaca ctttgtgggt ttgaacagat 420
gtataatgac atggagtcac cattgcagca tcatagagta gtttcaccag cccccagatc 480
ccccaaatcc gctgtgctca gcttgctcat ccctggcttc ccctaaccca gggcgaccac 540
tggtcttttt actaccttta tagttttgcc atttccaaaa tgttgctgag ttgaatgaca 600
cagtatatag ccttttcaga ttggcttctt taacctggca atatgcattg aatgttactc 660
catgtttttg catgatgata gttcacttcc ttttttcagc agtgtataat attccatcat 720
ctggatgtac cgccgtttat ttatccatgt cactgaagga catcttggtt gctcccaagt 780
tttggaagtt atgagtaagg ctgctataaa catctgtgga taggttttta tctggacata 840
ttttaacgct gataaatacc aaggagtgtg attactggat agtatggtaa gcatatgttt 900
aattgggtag gaaattgcaa gctgtctcca cagtggctgt accattttgc aattccacca 960
gtaaaagatg agagtccctg ttgctccaca tcctctccag catgttggtg ttgtcagtgg 1020
gtttmggcca ttccactgag tgtgcagtcg agtcttattg ttttaacttc catttccctg 1080

aggacctatg acgtggagca tcttctccta tgcgtgtttg ccagctgtag atgttctttg 1140

atgaggtgtc tgttcaggtc cttggcgcat tttttaatcc ggttgtttgt tttcttattg 1200

ttgagtttta ggacttcttt gtatatattt tggataacag tcctttatca gatgtgtatt 1260

ttgcaaatat atcctcccag tgagtggctt gtcttggcag tgtttttcac agagcagaag 1320

ttgttaattt gtttgaagtt cagctaatca gtcctttcac ggactgtgcc tttggtgttt 1380

tatctaaaac atcatggttg tgcccaaggt ggccaagatt ttcccctctg atatcttcca 1440

gtttagagtt tgccttttgt ggttatgtct aggatccatt ttgagtaaag ttttgtgatg 1500

ggtgtaaggt ctgtgtctag atcctgcccc cccccacaca ctttctttgc ctgtggatgt 1560

ctggttgttc cagcaccatt tgttgaaaaa tgatcttttc tctactgagt tgcctttgcc 1620

cctttgtcaa agatgggttg actgcatctg ggtgggtctg tttctgagct ctgtgttctg 1680

ttcccttgat ccatttgttc tttctttcac taatgtcaca ctgtattgat taccatggtt 1740

ggcagtaact cttcaatgtt tactactttt aatacactaa atacagttcc aagactatgg 1800

gtttttaaag ctctaaaata tcttttattc taaatagttt aaactgtatc ctttttg 1857

<210> 6
<211> 601
<212> DNA
<213> Homo sapiens

<220>
<221> variation
<222> (301)..(301)
<223> SNP rs11037653

<400> 6
taagaaaaat aaatcaccag ggtacacccc atttgtttga agtgttaagc accaaacagt 60

cagtgcttaa taaagaaaat caatattttc agaattacaa aggaaaatgc taaagccctt 120

agtcatgaaa gaaacattaa gttttcagta agataagtgg aaaggaaaca gccatgaatt 180

gtgagaattt atcctgaaag gtcaccataa cttgatgaga ctataaatgc ttaaggttga 240

gaagttggat tcttaactcc gttacttaaa aaaaaactaa gcatctactg tcttctttca 300

rgcaaatgcc aagaaataca gatttcagaa agcttataaa actttggcta tttagagaaa 360

atgacatcct tactgctttg agtaagcaaa caattggtta acaaagtgtg attactgtaa 420

tgcttacttg attccttagg agagactctc atgtgtgctc cagattggtt tggaggactt 480

tgcacaatac aactcaggga ggcgtaattt accaaggcca aaagagtagg cttggctttt 540

aagtatgcca cagacaatac ttttttcaaa ttctactact atgacttttt taactttgtg 600

a 601

<210> 7
<211> 601
<212> DNA
<213> Homo sapiens

<220>
<221> variation
<222> (301)..(301)
<223> SNP rs4620711

<400> 7
tcacaacaat tttgtaaaga tttgggaaag aataataaat atactaccaa atttatattt 60

aatgtttatt aaatatggca gagtgttcag ttccctgtaa ctccctgtaa tttcctgata 120

ccacagatga agaaaggtta gcctgttacc aatactattc aaggacatct ttttgatctc 180

agtgcactat acattttcaa aactagtatg acagccctgc tatcataact ctgtaaccag 240

ctttgtctaa ccagccttgg tttattttca atctagcatt tttagatgta tagcacttga 300

rgtttcagaa gaaggagaaa aaacagttca caaattattt tgcaaagtag aacaaaaaat 360

agaaaactaa agcaaaacat ctgaccccccc aattgaaata caccagaacc tcaaggaaaa 420

aagtattcac tcatgaaata ttatagtgca aatacacttt tgagcaaatg aagtgataca 480

tgcacacata tacatacaca cgactaataa ggtaaactgt gaaaaagccc accagaatga 540

atacaagaaa tactattaga gttccaaaag tgcacagtca ctatcatcta gaaagctctc 600

a 601

<210> 8
<211> 3723
<212> DNA
<213> Homo sapiens

<220>
<221> variation
<222> (1723)..(1723)
<223> SNP rs10838166

<400> 8
agcatttaat cagcctgtat tatgcagcat agatgtcaaa gatacttgaa gggatttcca 60

ctatccagct tgaagggatg ctatctaacc aaacctggat aaattgagca ttaaaattaa 120

aaggatggaa acaaattata atctgttaat taaaatagaa aaccatggcc gggtacggtg 180

gctcactcct gtaatcccaa cactttgaga gcccaaggca ggaggattac ctgaggtcag 240

gagttcaaga tgagcctggt caacatagtg taaccccgtt tactaaaact acaaaagtta 300

gctgggtgtg gcacgagcct gtagtcccag ctacttgtga ggctgaggca ggagaattgc 360

ttgaaccggg aggcaaaggt tgcagtgagc caagattgcg ccactgcact ccagcctggg 420

tggcagagag agactctgtc tcaaaaaaaa aaaaaaaata ggaaagcatg agcccattgt 480

aattatgata agtaaatgaa tgaaatgaaa tttttacaag gaatgggatt taatgtagtc 540

ttaaagtatc ttcctacaga atacaaaaac ttgcaaatta aaaaggaaat aatagtaact 600

tttatagtgg aggaaactgg cagacactac cttaataaga gacaaaagtt atcatcatca 660

gtactgggac aaatcaaaat gatgtgccac ctgatgggat gcagtgagaa acacagcatc 720

acttctgtga tattctggcc aaaagtgcat atcctgaatc taatcatgag gaagcataca 780

aacctaagtt gagggacatt ctacaaagta attggcctgt aataatcaat gtatcaagga 840

```
tatgaaggtc aaggacaggg cagaaagact gttccagatt gaaggaggct aaccagaatg     900
gtagctaaat gcattgggtg attctgactt ggatcctttt gcagtaaagg atattgttgg     960
gacaactagt aaaaattgac aggctctgag gattagaaga tagtaattat tcatcaatag    1020
taattattcc tcaatgttaa tttcttgatt tcgatggtta catcatttag gagactgcag    1080
taagtacata ctaaggcagc attctcaaac tttttagtct cagagccctt tttactctta    1140
aatattattg aggactccaa agagcttttt tttttttttt actttttttt ttaaattata    1200
ctttaagttc tgggatacac gtgcagaaca tgcaagtttg ttacataggt atacacgtgc    1260
catggtggtt tgttccaccc atcaactcgt aatctacatt aggtatttct cctaatgcta    1320
tccctctgct tgccacccac accctgaaca ggccctggtg tgtgatgcgc ccttccctgt    1380
gtccatgtgt tctcattctc attgttcaac tcccacttat gagtgagagc atgcggtgtt    1440
tggttttctg ttcttgtgtt agtttgctga gaatgatggt ttccagcgtc atccatgtcc    1500
ctgcaaagga catgaacaca tccccaaaga gcgtttgttt acataggata ccactatcaa    1560
tgtttatcac attataaatt gaaactgaga aacttaaaaa ataattattt aactcactta    1620
aaaataataa cagtaaaccc tttacatgtt aacactacac attttgaggg aaaataacta    1680
tatattctga aacaaaacaa aatatagtga gaacaaccac atsattttac atttacaaaa    1740
tctctttaat atctggcatt gtagcaggat gagccacaga caaaactcct cagacaccgg    1800
attaaagaag gaagaggatt tattcggcca ggagcatcag cagacttgcg tcttaagagc    1860
tgagctccct caaaaagaaa ttcttggccc ttttaagggc ttacaactcc taaggggtcc    1920
acatgaaaga gtcatgatag atcgagtaag catgggaaac gttactgggg gctacatgca    1980
taagctaaca gaacagaaag ttttgcaatg ctttttcata caatgtctgg aatttacaga    2040
taacacaagt agtttaggtc agggattgat attattatta ttattattat tattattatt    2100
attattatta ttattttaac tgccagggcc agatggtgac accaggccat ctggctattt    2160
atcttctttt taactttttg ctttttttctt ttcctcctgt cttataaact agacaagggc    2220
aggtgggggt gggggggcagc aggagaaatg gtggtctcct tccttattcc ccccttggag    2280
aatttcactt attagttgaa gttctcactt ttatttttac tttctgagtc tctttgtgag    2340
acagagtgat agtgattcat gtaatacact tgtgctgaag ttttctgatg aaccagggtg    2400
gcaacaaaac cttttatcat ttgaaggagc aagggtaata cgcaggggag cagcaagcaa    2460
gttcttatta ctagcagtat acttacaatg agggttttaa atcttcttat agctggaaac    2520
tatttttcta aataaagact taggattaaa cctgtgctaa acctgtacag acacatgtgc    2580
caactttatc atgttctaag caggtttgtt cttttactgg gtcttaaaag ccttttttta    2640
gtgagcagca tagtatttct taataacaga actttttaaa cgccagacac tggaacttgt    2700
gagcatctgt ttggggaaag agtcagttaa attaaagtta tcttgaggca ttaacttttt    2760
tgcttcttaa ggctattggt ctctcatgtt agcttttcta taaacataac atgaggaaat    2820
gcttaggctg ctgacagtgt tttcagccag ctaagtaaac agggtttttt tttttgctaa    2880
atgaggaggc tctggtaact tctggtttat atgcttaaag aatgacttaa agactcagaa    2940
```

```
ttgttgctgg gccagacagg ttcgggttac ttgaccaagt agtgtgtgta cagtggggac    3000
atctttctat aggtgtttct tctagcatgg ttaaggggga taacaacaac aaaacaatat    3060
acagcatatt catatctagc aaggacaaaa gaggtcctta cctgggaaaa aggttgaata    3120
cagcgacaga acaataggaa aacagttaat attacatgaa aactattagt cttaagattt    3180
ttaactacat ttacttgctt gatgagtcct caagctttgg ctgtgcatag actagtcagc    3240
ttccaggatg tgactagagc agagcttgct ctagtccaaa cagtttagtc taaaacatag    3300
ttagatttgc atatctactt ctgcattcag tctgctgcaa tttgttattt tggttgaagt    3360
agaaggaaat ctggctttac atggatgcgt agttggagaa gagaaagcta ttttaatagc    3420
ttctttggat gattgtggat attcttttgg tccatcaaaa cattctaagt gaaagcttct    3480
taaaagttag ttgcaatatg gaatctgaaa ttatatcaat tttttacact gagtgacatt    3540
aaaatattaa agttcattgt tctacctcat agtctgaata ggtcttttat ccatcatgac    3600
tttgtaactt tatgcattga tcacttggaa aatatttgtt cctgaatttt gcagctcttc    3660
caaatgttga cacagtatca aagataatca caattgttaa tattaccccc aatcttatca    3720
gaa                                                                  3723
```

<210> 9
<211> 835
<212> DNA
<213> Homo sapiens

<220>
<221> variation
<222> (501)..(501)
<223> SNP rs4573056

<400> 9

```
gcatcatgct atcagcccat agcaatagac attataatac aaactttcag gaaaaaaatt      60
tatgtgtgtc tatgtatata ttattgaata tatttttaag ttgtcatgtc aattatatta     120
ccttaaaaat tcaaaaatta agtgcataag acaacgctta ttttattata tttactgatc     180
ctctgggtca aaaatttaaa tattgcacaa tgatacttct ctccttttca taatatcttg     240
tactacaact gtgaagacct aaataattgg agtgattcaa aaagctgaga gctagaaaga     300
tctggagact tcttttcaca tatttgtgtt gcctggtttt atgtgactta aagaatgagc     360
tcagctggag ttattggcca gaatgcccca tgtgaaaaat ttgccatgtg gcttggtgtt     420
cctcatagca tggttgtctc agggtagtca tgtttcatat gatggctcag ggctccaaga     480
gggagcattc cagtgaataa mgtgtagttt tcgtgtgctt tcctaatata gctttaagaa     540
tcatgcgtga ctatctctgt cacatgctat tagatgtagt aatttactaa gaccaggcca     600
tactcaatgt taggagaatt agacctgacc tcttacctga ggtaagaggt caggtctatt     660
tctgtcagaa atagaaatgt tttttattct gtcaagtgat tcaagtttta ttcatgaatt     720
atataactgc taaataccta cttttgcaaa aactattcag catatttctt gtaagtacat     780
agaaattcat agaatatatt ttgtgtacaa tattttgtaa gagaaccaac atatt          835
```

<210> 10
<211> 601
<212> DNA
<213> Homo sapiens

<220>
<221> variation
<222> (301)..(301)
<223> SNP rs10755496

<400> 10
gtgtcttaag acctgaggac tgtaaaatga taggatgaat ccatctaaat gatcagacag 60

aatagttttg tgtgactgtt ttttgcatgg gattattgtt gtcattttgc ttattcattt 120

tggactgaga aattaacttt gtgtttcttt tgcttattga atccatggga ttaaagatcc 180

agcttgatta aatggacatg gaatttttga tgtttggaag ataagtattg ttcataccaa 240

aaatcagaaa ttatctatca ttgtgtaact agttgcacat ttctcctaac ccttgtttta 300

waagtcatcc tctaaactgt taggaggaaa ataatccctt ctctggaaac tttatccttt 360

acagtgttta gcacttaaat gcaattaagc aaacataata tcctctttga agcaaaatag 420

ttgctcagct taagtaactg aaactaatta tctctgttga atgaaagaca tattcggcaa 480

gtgtttgaag aatgcttcct tcatgttgag cagaaattag cacttttatt ggcgttgcag 540

gtggaggagc actcaacatg ctgtatctgt cagttcatac ccacatgtag acctacaagt 600

a 601

<210> 11
<211> 861
<212> DNA
<213> Homo sapiens

<220>
<221> variation
<222> (201)..(201)
<223> SNP rs7742750

<400> 11
ttgaaccaat gacttccaaa catgcctata atatttaagt gtgcagcttc ctgtacctac 60

ctcaggccca gtgtataaga tcctggaaat ctgtatttat tattcccata gtgactatat 120

tattaggaaa gtcttagaaa cactaggtaa accagacatt caaagagctt ggtattcaga 180

ttatgacaat tcaacctttg rtacaaaata tttaggcatc atgctatcag cccatagcaa 240

tagacattat aatacaaact ttcaggaaaa aaatttatgt gtgtctatgt atatattatt 300

gaatatattt ttaagttgtc atgtcaatta tattacctta aaaattcaaa aattaagtgc 360

ataagacaac gcttatttta ttatatttac tgatcctctg ggtcaaaaat ttaaatattg 420

cacaatgata cttctctcct tttcataata tcttgtacta caactgtgaa gacctaaata 480

attggagtga ttcaaaaagc tgagagctag aaagatctgg agacttcttt tcacatattt 540

gtgttgcctg gttttatgtg acttaaagaa tgagctcagc tggagttatt ggccagaatg 600

```
ccccatgtga aaaatttgcc atgtggcttg gtgttcctca tagcatggtt gtctcagggt    660
agtcatgttt catatgatgg ctcagggctc caagagggag cattccagtg aataaagtgt    720
agttttcgtg tgctttccta atatagcttt aagaatcatg cgtgactatc tctgtcacat    780
gctattagat gtagtaattt actaagacca ggccatactc aatgttagga gaattagacc    840
tgacctctta cctgaggtaa g                                              861
```